(19) Europäisches Patentamt
European Patent Office
Office européen des brevets

(11) **EP 4 102 509 A1**

(12) **EUROPEAN PATENT APPLICATION**

(43) Date of publication:
**14.12.2022  Bulletin 2022/50**

(51) International Patent Classification (IPC):
**G16C 20/20** $^{(2019.01)}$

(21) Application number: **21178053.1**

(52) Cooperative Patent Classification (CPC):
**H01J 49/0036; G16C 20/20**

(22) Date of filing: **07.06.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **MSAID GmbH**
**85748 Munich (DE)**

(72) Inventors:
• **WILHELM, Mathias**
  **85354 Freising (DE)**
• **FREJNO, Martin**
  **50767 Köln (DE)**

(74) Representative: **Boult Wade Tennant LLP**
**Salisbury Square House**
**8 Salisbury Square**
**London EC4Y 8AP (GB)**

(54) **METHOD AND APPARATUS FOR IDENTIFYING MOLECULAR SPECIES IN A MASS SPECTRUM**

(57)    There is provided a method of identifying one or more molecular species represented in a mass spectrum. The method comprises obtaining a set of candidate mass spectra for the mass spectrum, wherein each candidate mass spectrum corresponds to a respective candidate molecular species; optimizing a set of mass spectra coefficients for the set of candidate mass spectra, based on the mass spectrum; and providing, for one or more of the candidate molecular species, a respective indication of a match in the mass spectrum, based at least in part on the optimized set of mass spectra coefficients. Said optimizing comprises: varying the mass spectra coefficient values based on an objective function, wherein the objective function relates a linear combination of the candidate mass spectra, according to the mass spectra coefficients, to the mass spectrum, subject to a regularization term of the objective function constraining the number of non-zero mass spectra coefficient values. There are also provided corresponding systems and computer programs.

Fig 3

EP 4 102 509 A1

**Description**

**Field of the invention**

The present invention relates to identification of molecular species present in mass spectra. In particular methods and systems for identifying molecular species in chimeric mass spectra.

**Background of the invention**

The use of mass spectrometry (MS) techniques has become invaluable across many fields where detailed analysis of various chemical, and often biological, samples is required. Such mass spectrometry analysis is used to identify the chemical makeup of given samples.

Straightforward analysis in a mass spectrometer typically involves the generation of ions from a chemical sample. The mass-to-charge ratio (m/z) and abundance of these ions are then measured by the mass spectrometer to produce a mass spectrum. The peaks (or centroids) in intensity at particular m/z values in such a mass spectrum provides a signature that indicates the relative abundance and m/z of respective ions. This signature may allow the compound (or compounds) that make up the original chemical sample to be identified.

Often for samples including proteins and the like identification can be done by automatic searching of libraries of known mass spectra for known compounds and seeking to match the relative intensities at given m/z values (within a tolerance) in the known mass spectra to the relative intensities at the same m/z values in the measured mass spectra. Where the pattern of relative intensities match between the two mass spectra this indicates that the compound corresponding to the library mass spectrum is present.

Similarly a database searching approach may be used where the experimental spectra are compared to calculated peptide fragment mass lists derived from protein databases. Such approaches have become an important tool for the identification and quantification of proteins and their modifications in samples. Such identification is particularly useful with the technique of mass spectrometry-based bottom-up proteomics.

In bottom-up proteomics usually proteins are extracted from their origin (e.g. tissue) and might be proteolytically digested using a peptidase (usually with a specific cleavage pattern) to obtain shorter poly-amino-acid chains termed peptides. After digestion, samples may undergo further processes such as: sample clean-up in order to remove unwanted salts or other reagents from the sample, offline fractionation (e.g. liquid chromatography) to decrease sample complexity, enrichment of specific classes of (modified) peptides using physicochemical properties or antibodies, or labelling using isobaric labelling reagents (e.g. tandem mass tags TMT, TMTpro, iTRAQ).

The resulting peptide mixtures may then be separated based on their physicochemical properties. For example a reverse phase liquid chromatography system which is directly coupled to a mass spectrometer may be used.

Peptides that elute from the liquid chromatograph are usually ionized into the gas phase using electrospray ionization (ESI). The mass spectrometer measures mass-to-charge ratios (m/z) for the peptide ion (or precursor ion). Two modes of operation can be distinguished: MS1 and MS2/MSn-scans. MS1 or full-MS scans record the m/z of intact precursor ions, MS2/MSn scans isolate a certain ion population (e.g. a specific m/z range of the MS1 scan) for subsequent fragmentation (e.g. via collision with neutral gas molecules) and record the m/z of the resulting fragment ions. The specific m/z values and relative intensities of the fragment ions allows the amino acid sequence to be determined. It is often assumed that the MS2/MSn spectra should contain fragments originating from a single peptide (or precursor). However, dependent on the width of the isolation window (tolerance for selecting ions for a subsequent MS2/MSn scan) more than one precursor ion can be co-isolated and co-fragmented in one MS2/MSn event leading to chimeric MS2/MSn-scans containing fragment ions from multiple precursor ions. Indeed, for more complex measured samples, such as non-fractionated cell lysate on a short gradient, a larger number of precursors may elute per unit time such that more precursor ions co-elute and are co-isolated in each MS2/MSn isolation window. This results in an increased likelihood that the resulting MS2/MSn spectra include more than one precursor ion.

In a mass spectrum that is generated from a sample containing more than one compound (such as an MS2 scan having more than one precursor molecular species, or even an MS1 scan having more than one molecular species present) there may be multiple compounds contributing to the intensity of a given peak. This can make identifying the molecular species from the resulting mass spectrum difficult. Whilst there are a number of existing methods aimed at attempting to identify the contributions of individual molecular species to a given mass spectrum, such as: Complementary ion boosting, outlined in "Deconvolution of Mixture Spectra and Increased Throughput of Peptide Identification by Utilization of Intensified Complementary Ions Formed in Tandem Mass Spectrometry", Fedor Kryuchkov, et al., Journal of Proteome Research, 12 (7), pp. 3362-3371, (2013) DOI: 10.1021/pr400210m; and DIA-Umpire, outlined in "DIA-Umpire: comprehensive computational framework for data-independent acquisition proteomics", Tsou, CC. et al. Nature Methods, 12, 258-264 (2015) DOI: 10.1038/nmeth.3255, these methods typically are limited to cases where the mass spectrum is from an MS2 scan, and part of a series of mass spectra from a chromatography experiment. They are then able to use inferred chromatographic features (such as elution peaks) to aid in the identification of precursor ions (typically peptides in proteomics experiments).

## Summary of the invention

It is an aim of the invention to provide improved systems and methods for identification of molecular species in a mass spectrum where multiple molecular species may be present without the limitations of the systems of the prior art. Whilst such systems and methods may be particular advantageous for use in the field of proteomics it will be appreciated from the discussion below that they are not limited to such a field and are generally applicable to mass spectrometry experiments.

In the present invention new methods and systems for identifying one or more molecular species represented in a mass spectrum are proposed. The invention provides a method for identifying one or more molecular species represented in a mass spectrum by regularized regression of the mass spectrum using selected candidate mass spectra corresponding to candidate molecular species.

In a first aspect there is provided a method (such as a computer implemented method) of identifying one or more molecular species represented in mass spectrometry data (such as or in the form of a mass spectrum).

In this aspect the method comprises obtaining a set of candidate mass spectra (or items of candidate mass spectrometry data) for the mass spectrum (or mass spectrometry data), wherein each candidate mass spectrum (or item of candidate mass spectrometry data) corresponds to a respective candidate molecular species. The method continues with a step of optimizing a set of mass spectra coefficients for the set of candidate mass spectra, based on the mass spectrum. Said step of optimizing comprises varying the mass spectra coefficient values based on an objective function. The objective function relates (or comprises a term relating) a linear combination of the candidate mass spectra, according to the mass spectra coefficients, to the mass spectrum. The objective function may comprise a term that is a function of the difference (or deviation or error) between the linear combination of the candidate mass spectra according to the mass spectra coefficients and the mass spectrum. In some embodiments the term may be a mean squared error between the linear combination of the candidate mass spectra and the mass spectrum.

The optimization is subject to a regularization term of the objective function constraining the number of non-zero mass spectra coefficient values. The regularization term may comprise a norm (such as an $L_1$ norm) of the mass spectra coefficient values. The regularization term may be arranged to enforce sparsity in a vector formed from the coefficient values. In some embodiments the optimizing may further comprise varying a degree of regularization of the regularization term (for example by varying a regularization parameter of the regularization term).

The method also comprises providing, for one or more of the candidate molecular species, a respective indication of a match in the mass spectrum, based at least in part on the optimized set of mass spectra coefficients. The respective indication may comprise a quantity (such as a predicted or estimated quantity) of the corresponding candidate present in the mass spectrum.

The method may further comprise identifying the mass spectrum as a chimeric mass spectrum based on the optimized set of mass spectra coefficients.

In some embodiments the one or more molecular species are one or more precursor molecular species, the mass spectrum is a fragment mass spectrum derived from the one or more precursor molecular species, and each candidate mass spectrum is a candidate fragment mass spectrum corresponding to a respective candidate molecular species. The precursor molecular species represented in one or more candidate fragment mass spectra may be peptides.

The step of providing may comprise identifying the one or more candidate molecular species as sample molecular species represented in a chimeric mass spectrum based on the optimized set of fragment mass spectra coefficients.

In some further embodiments for a given candidate mass spectrum a spectrum similarity score is calculated between the given candidate mass spectrum and a further mass spectrum. The further mass spectrum being generated by subtracting each of the other candidate mass spectra from the mass spectrum according to the optimized set of mass spectra coefficients.

In some embodiments the mass spectrum is an MS1 mass spectrum of the one or more molecular species and each candidate mass spectrum comprises an isotope pattern corresponding to a respective candidate molecular species.

In some embodiments the one or more molecular species are a plurality of isobarically labelled (such as with Tandem Mass Tags) precursor molecular species, the mass spectrum is a fragment mass spectrum derived from the plurality of isobarically labelled precursor molecular species, and each candidate mass spectrum is a candidate fragment mass spectrum corresponding to a respective candidate isobarically labelled molecular species. The step of providing further comprises generating reporter ion intensities for one or more of candidate mass spectra and generating corrected reporter ion intensities for at least one of the precursor molecular species based on a difference between a reporter ion intensity for the precursor molecular species generated from the mass spectrum and the reporter ion intensities for the one or more candidate mass spectra scaled by the corresponding optimized mass spectra coefficients.

In some embodiments the mass spectrum is an MS1 mass spectrum and wherein one or more of the candidate mass spectra are selected as part of the set of candidate mass spectra based on an MS2 mass spectrum corresponding to the MS1 mass spectrum.

In some embodiments the mass spectrum is an MSn (such as an MS2) mass spectrum and wherein one or more of the candidate mass spectra are selected as part of the set of candidate mass spectra based on another mass spectrum at the same or a different mass spectrum level (such as MSn, MSn+1 or MSn-1) corresponding to the MSn mass spectrum.

In some embodiments the mass spectrum is part of a series of mass spectra for a separation (such as a chromatographic, m/z, mass or ion mobility separation) wherein the regularization term comprises a constraint enforcing a relation between the mass spectra coefficient for a given candidate mass spectrum and a mass spectra coefficient of the same candidate mass spectrum determined for further mass spectra of the series (such as further mass spectra adjacent to the mass spectrum in said series).

In some embodiments the objective function is arranged to provide a measure of difference between the linear combination of the candidate mass spectra and the mass spectrum. In some further embodiments the step of varying is carried out with the aim of obtaining an extremum value of the objective function.

The invention also provides apparatus corresponding to, and comprising elements, modules or components arranged to put into effect the above methods, for example one or more various suitably configured computing devices such as those described previously.

In particular the invention therefore provides a system (or apparatus) for identifying one or more molecular species represented in mass spectrometry data (such as or in the form of a mass spectrum). The system comprises a candidate selection module arranged to obtain a set of candidate mass spectra (or items of candidate mass spectrometry data) for the mass spectrum (or mass spectrometry data), wherein each candidate mass spectrum (or item of candidate mass spectrometry data) corresponds to a respective candidate molecular species.

The system further comprises an optimization module arranged to optimize a set of mass spectra coefficients for the set of candidate mass spectra, based on the mass spectrum. Said optimizing comprises varying the mass spectra coefficient values based on an objective function. The objective function relates (or comprises a term relating) a linear combination of the candidate mass spectra, according to the mass spectra coefficients, to the mass spectrum. The objective function may comprise a term that is a function of the difference (or deviation or error) between the linear combination of the candidate mass spectra according to the mass spectra coefficients and the mass spectrum. In some embodiments the term may be a mean squared error between the linear combination of the candidate mass spectra and the mass spectrum. The optimization is subject to a regularization term of the objective function constraining the number of non-zero mass spectra coefficient values. The regularization term may comprise a norm (such as an $L_1$ norm) of the mass spectra coefficient values. The regularization term may be arranged to enforce sparsity in a vector formed from the coefficient values. In some embodiments the optimizing may further comprise varying a degree of regularization of the regularization term (for example by varying a regularization parameter of the regularization term).

The system also comprises an indication module arranged to provide, for one or more of the candidate molecular species, a respective indication of a match in the mass spectrum, based at least in part on the optimized set of mass spectra coefficients. The respective indication may comprise a quantity (such as a predicted or estimated quantity) of the corresponding candidate present in the mass spectrum.

The invention also provides one or more computer programs suitable for execution by one or more processors, such computer program(s) being arranged to put into effect the methods outlined above and described herein. The invention also provides one or more computer readable media, and/or data signals carried over a network, which comprise (or store thereon) such one or more computer programs.

## Brief description of the drawings

Embodiments of the invention will now be described, by way of example only, with reference to the accompanying drawings, in which:

Figure 1a schematically illustrates an example scenario in which a sample is analysed using a mass spectrometer arrangement;
Figure 1b schematically illustrates a further example scenario in which a sample is analysed using a first mass spectrometer arrangement and a second mass spectrometer arrangement;
Figure 1c schematically illustrates an example mass spectrum such as may be produced by any of the mass spectrometry arrangements (or stages) described in relation to figures 1a and 1b;
Figure 2 schematically illustrates an example of a computer system which may be used in the invention;
Figure 3 schematically illustrates a logical arrangement of an example analysis system that may be used to analyze mass spectrometry data, such as the mass spectrometry data discussed above in relation to figures 1a-1c;

Figure 4 is a flow diagram schematically illustrating an example method of identifying one or more molecular species represented in received mass spectrometry data, such as may be carried out by the system of figure 3;

Figure 5 is a flow diagram schematically illustrating a further example of the step of optimizing such as the step of optimizing of the method shown in figure 4;

Figure 6 schematically illustrates an exemplary analysis system according to one embodiment of the invention;

Figure 7 shows an example experimental mass spectrum along with the linear combination of candidate mass spectra as calculated by an embodiment of the invention.

## Detailed description of embodiments of the invention

In the description that follows and in the figures, certain embodiments of the invention are described. However, it will be appreciated that the invention is not limited to the embodiments that are described and that some embodiments may not include all of the features that are described below. It will be evident, however, that various modifications and changes may be made herein without departing from the broader spirit and scope of the invention as set forth in the appended claims.

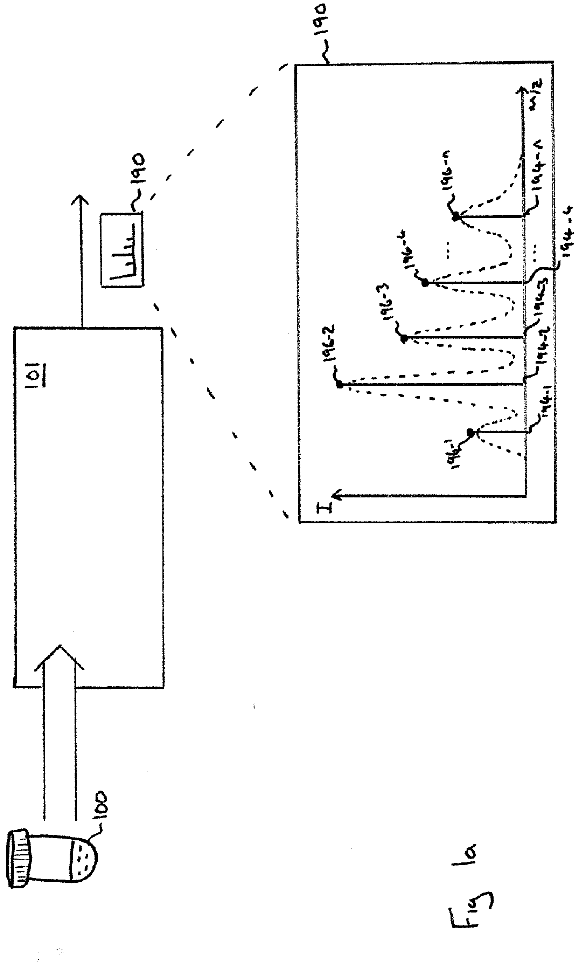

Figure 1a schematically illustrates an example system in which a sample 100 is analysed using a mass spectrometer arrangement 101.

The sample 100 comprises molecules of one or more molecular species. The sample 100 may be obtained directly. Additionally, or alternatively, the sample 100 may be obtained from the output of a separation technique, such as liquid (or gas) chromatography, ion mobility separation etc. as described shortly below. As shown in figure 1a the sample 100 is provided to the mass spectrometer arrangement 101.

The mass spectrometer arrangement 101 is arranged to receive the sample 100. The mass spectrometer arrangement may be arranged to ionize the sample 100 to produce ions of the one or more molecular species. Alternatively, the sample 100 may already have been ionized, for example as part of a separation technique used to obtain the sample. In some examples, as discussed further below, the mass spectrometer arrangements 101 may be arranged to fragment the one or more molecular species in the sample 100, such that the sample 100 comprises a plurality of fragment ions for each molecular species.

The mass spectrometer arrangement 101 is arranged to separate the ions (or fragment ions) of the sample 100 and measure the quantity (or relative abundance) of ions (or fragment ions) present in the sample as a function of the ions (or fragment ions) mass to charge (m/z) ratio. The mass spectrometer arrangement 101 may be arranged to output the measurement of the ions as mass spectrometry data 190, such as in the form of a mass spectrum. The operation of such mass spectrometer arrangements 101 is well known in the art and not described further herein. The skilled person would appreciate that the mass spectrometer arrangement 101 may be of any type. For example, the mass spectrometer arrangement may comprise any one of: a time of flight (TOF) mass spectrometer, a Fourier transform ion cyclotron resonance mass spectrometer (FT-ICRMS), an Orbitrap™ mass spectrometer etc.

An example graphical representation of mass spectrometry data 190 in the form of a mass spectrum is also shown in figure 1a.

The mass spectrometry data 190 can be represented in the form of one or more mass channels. Each mass channel corresponds to a respective mass value (or mass to charge ratio, referred to herein as m/z value) at which ionic species of the same mass (or mass to charge ratio) are detected in a mass spectrometer arrangement 101. Each m/z value corresponds to a respective ionic species and is equal to the molecular mass of the respective ionic species divided by the absolute elemental charge of the respective ionic species. The mass spectrometry data 190 comprises one or more intensity values 196-n with each intensity value 196-n appearing for a respective m/z value (or channel) 194-n. Each intensity value 196-n correlates to the relative abundance (or quantity) of the ionic species corresponding to the respective m/z value 194-n as measured by the mass spectrometer arrangement 101 from the sample 100. Each intensity value 196-n may be proportional to the relative abundance of the ionic species corresponding to the respective m/z value. The intensity (or relative abundance) measured by the mass spectrometer arrangement 101 for a given mass channel correlates to the relative abundance of ionic species having the mass (or mass-to-charge ration m/z) detected by the mass spectrometer arrangement 101. It will be appreciated that mass spectrometry data 190 (and by extension mass spectra) may be represented in any one of a number of different forms, such as a transient signal produced by an Orbitrap™ mass spectrometer or other Fourier-transform mass spectrometer such as an ion cyclotron resonance (FT-ICR) spectrometer, ion flight times produced by a time of flight (TOF) mass spectrometer, and so on. It will be appreciated that the methods and systems described below can be used with any representation of mass spectrometry data 190 (or mass spectra).

It will be appreciated that there may be more than one ionic species detected in a given mass spectrometry experiment with the same mass (or m/z value). As such the intensity value for a given mass channel may represent the sum of the abundances of all of the ionic species detected in a given mass spectrometry experiment with the mass (or m/z value) corresponding to that particular mass channel, as described shortly below.

Experimental mass spectrometry data such as the mass spectrometry data 190 may be plotted in the form of a continuum plot, indicated by the dashed line, and a centroid plot, indicated by the vertical solid lines. The widths of peaks indicated by the dashed line represent the limit of the mass resolving power, which is the ability to distinguish two different ionic species with close m/z ratios.

However it will be appreciated that the mass spectrometry data 190 does not need to be plotted in the form of a graph. Indeed, the mass spectrometry data 190 may be represented in any suitable form. For example, the mass spectrometry data 190 may be represented a list comprising the one or more intensity values 196-n and the one or more m/z values 194-n. In some cases the mass spectrum may simply be represented as a list of centroids (or local maxima), each centroid being represented as an m/z value and intensity value pair.

As there are many techniques commonly used in the art for obtaining such centroids from mass spectrometry data these will not be discussed further herein. However, it will be appreciated that the techniques described herein may be performed on lists of centroids forming mass spectrometry data, or on raw mass spectrometry data where suitable techniques are used to identify the intensity maxima (or centroids).

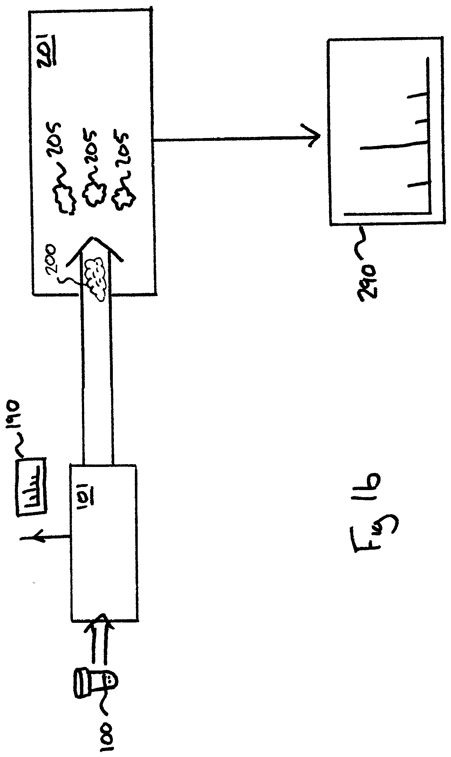

Figure 1b schematically illustrates a further example in which a sample 100 is analysed using tandem mass spectrometry. For ease of understanding figure 1b shows a first mass spectrometer arrangement 101 and a second mass spectrometer arrangement 201. However it will be understood that in tandem mass spectrometry these typically correspond to a single mass spectrometer arrangement (such as described above) operating in a first mode 101 and a second mode 201 respectively. The discussion above of the mass spectrometer arrangement 101 in relation to figure 1a applies equally to these first 101 and second 201 mass spectrometer arrangements.

In this further example an initial sample 100 is provided to the first mass spectrometer arrangement 101 (or first operating mode), as described above in relation to figure 1a. The initial sample 100 comprises ions of one or more molecular species, which are separated and measured by the first mass spectrometer arrangement 101 to generate an initial mass spectrometry data 190 for the initial sample 100. The second mass spectrometer arrangement 201 (or second operating mode) is arranged to operate such that a mass filter of the mass spectrometer arrangement is switched to a mass-selective mode to select a subset of the ions in the initial sample 100 having a m/z ratio within a predefined range. The subset of ions may be thought of as forming a further sample 200. This allows ions of different molecular species having similar m/z values to be isolated for further analysis as described below. It will be appreciated that the further sample 200 can alternatively or additionally be formed by selecting ions based on other physicochemical properties such as ion mobility.

The second mass spectrometer arrangement is arranged to fragment the ions in the further sample 200, such that the further sample comprises fragment ions 205 for each of the molecular species (known as precursor molecular species) originally present in the further sample 200. The second mass spectrometer arrangement 201 is arranged to separate the fragment ions 205 of the further sample 200 and measure the quantity of fragment ions 205 present in the sample as a function of the fragment ions 205 m/z ratio. In particular the second mass spectrometer arrangement is arranged to generate further mass spectrometry data 290 for the fragmented further sample 200.

It will be appreciated that this further example is an example of what is commonly known as tandem mass spectrometry (or MS/MS). In effect the second mass spectrometer arrangement 201 is a selective mode of operation of the first mass spectrometer arrangement 101 operable to select a further sample 200 from the initial sample 100. As the precursor ions of the molecular species of the further sample 200 are close in their mass to charge ratios the fragmentation of these precursor ions into fragment ions 205 of the further sample 200 allows discrimination between the precursor molecular species based on expected patterns of fragment ions 205 associated with each precursor molecular species. This is also advantageous as an accurate mass alone is often not enough to determine the exact structure of a molecular species. As such the second mass spectrometer arrangement 201 may be considered to provide further structural information based on the fragment ions. The mass spectrometer analysis of the first mass spectrometer arrangement 101 (or first operation mode) is commonly known as the MS1 stage and that of the second mass spectrometer arrangement 201 (or second operation mode) is known as the MS2 stage. It will be appreciated that further stages (MSn, where n=3, 4, 5, ...) can be added where the fragment ions 205 from a previous stage (such as an MS2 stage) are again selected based on m/z ratio and used as precursor ions for the next stage where they are further fragmented and analysed in a mass spectrometer arrangement 201. These stages, again, can be carried out by further operating modes of the same mass spectrometer arrangement. This, again, aids in discrimination between precursor fragment ions that are close in m/z ratio, and/or provides further structural information on a given precursor ion. In both cases the MS1 mass spectrometer arrangement 101 (or first mode of operation) and the MS2 (or MSn) mass spectrometer arrangement 201 (or second mode of operation) may be considered to be particular examples of the mass spectrometer arrangement 101 discussed above in relation to figure 1a. Examples of such tandem mass spectrometer arrangements that have a first and second mode of operation include the Orbitrap Q Exactive and Orbitrap Exploris instruments produced by Thermo Fisher Scientific GmbH of Bremen, Germany, which both comprise an orbital trapping type mass analyser in the mass spectrometry arrangement. The orbital trapping type mass analyser is described in detail in WO-A-02/078046, the entire contents of which are incorporated herein by reference, and will not be described in detail here.

The methods and systems of the invention as described herein below are generally applicable to any of the mass spectrometry stages and modes of operation described above.

It will be appreciated that the mass spectrometer arrangements 101; 201 above may also be used with separation devices such as a liquid (or gas) chromatograph. Such a separation device is configured to separate a master sample into a plurality of samples 100. In particular, the separation device is usually configured to cause components (or analytes) of the master sample to elute (or emit or otherwise emanate) from the separation device as a function of a separation parameter (or dimension). The separation parameter (or parameters) may also be thought of as an elution parameter, especially where the separation device comprises a chromatograph or chromatography column. For example, the separation device may be a liquid (or gas) chromatograph, of the types commonly known in the art. In this example the elution parameter would be retention time. In other words the duration of time needed for the component to pass through the chromatograph (e.g. the time between the sample being injected into the device and the component being provided to the mass spectrometry arrangement 101; 201). As liquid (and gas) chromatographs are well known in the art they will not be described further herein.

The analytes are typically emitted by the separation device as a stream of molecular species, which may then be introduced (or injected) into and ionized in the mass spectrometer arrangement 101; 201 (though it will be appreciated that separation ionization devices may be used in addition or in the alternative). As such, the samples 100 are typically received by the mass spectrometry arrangement 101; 201 as a function of the separation parameter. In this way it will be appreciated that the mass spectrometry arrangement 101; 201 receives samples (or analytes) emitted at the same value (or within the same range of values) of the elution parameter simultaneously (or substantially simultaneously). Consequently, the mass spectrometry arrangement 101; 201 may be arranged to generate multiple items of mass spectrometry data 190; 290 (such as in the form of multiple mass spectra) as a function of the separation parameter. In other words each item of mass spectrometry data 190; 290 (or each mass spectrum) is generated for a respective value of the elution parameter. More specifically, each item of mass spectrometry data 190; 290 may be thought of as (or representing) a mass spectrum of the sample 100 emitted at a respective value of the elution parameter.

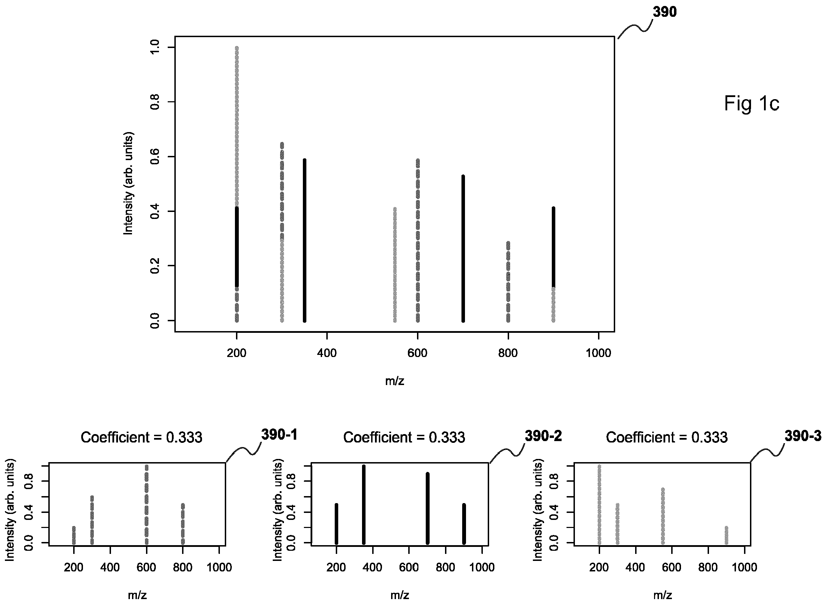

Figure 1c schematically illustrates example mass spectrometry data 390, in the form of a mass spectrum, such as may be produced by any of the mass spectrometry arrangements 101; 201 (or stages) described above in relation to figures 1a and 1b. It will be appreciated that the discussions of the mass spectrometry data 190; 290 above apply equally to this example mass spectrometry data 390, graphically represented in the form of a mass spectrum and vice versa. Figure 1c also schematically illustrates three further items of mass spectrometry data 390-1; 390-2; 390-3, graphically represented in the form of mass spectra.

The example mass spectrometry data 390 shown in figure 1c corresponds to a sample 100; 200 comprising a plurality of molecular species. As such the example mass spectrometry data 390, as generated by a mass spectrometer arrangement 101; 201 from the sample 100; 200, may comprise contributions from each of the molecular species present in the sample 100; 200.

The further mass spectra 390-1; 390-2; 390-3; each correspond to a respective molecular species of the plurality of molecular species in the sample 100; 200. In the example shown in figure 1c there are three molecular species in the sample 100; 200 and therefore three further mass spectra 390-1; 390-2; 390-3 are discussed. However, it will be appreciated that any number of molecular species may be present in the sample 100; 200. Each further mass spectrum 390-1; 390-2; 390-3; is a mass spectrum corresponding to (or representing) a sample comprising only the respective molecular species. In other words, each further mass spectrum 390-1; 390-2; 390-3; may be thought of as being (or representing) a mass spectrum that would be obtained from a mass spectrometer arrangement 101; 201 from a sample comprising only the respective molecular species. It will be appreciated that where such further mass spectra 390-1; 390-2; 390-3 are obtained from a mass spectrometer arrangement 101; 201 the respective sample may not be completely pure, however it will be understood that the presence of other molecular species in the respective sample would be at a level where the impact upon the further mass spectrum is negligible. In some examples one or more (or all) of the further mass spectra 390-1; 390-2; 390-3 may be generated by simulation (or numerical calculation).

As illustrated in figure 1c the example mass spectrum 390 may be thought of as comprising a sum of the further mass spectra 390-1; 390-2; 390-3. In other words the example mass spectrum may be made up (at least in part) from contributions of each of the further mass spectra corresponding to each molecular species in the sample 100; 200. The contribution from each further mass spectrum 390-1; 390-2; 390-3 is scaled by a corresponding scale factor (or coefficient) representing (or indicating) the proportion of the respective molecular species corresponding to the further mass spectrum present in the sample 100; 200. The further mass spectra are typically scaled by multiplying each intensity value in a further mass spectrum by the scale factor for that further mass spectrum.

As set out above mass spectrometry data may be thought of as a set of intensity values at particular mass-to-charge ratios (or mass channels). As such the example mass spectrometry data 390 may be thought of as having an intensity value at each mass-to-charge ratio (or mass channel) that comprises a sum of the intensity values of the further mass spectra 390-1; 390-2; 390-3 at each mass-to-charge ratio, scaled according to the relative abundance of the molecular species for each further mass spectra 390-1; 390-2; 390-3 in the sample 100; 200.

It will be appreciated that for an example mass spectrometry data 390 obtained from a mass spectrometer arrangement 101; 201 the example mass spectrometry data 390 may also comprise additional contributions to the intensity values corresponding to measurement error and/or random noise.

The mass spectrometry data described above may be thought of in terms of a mathematical function representing intensity, $I$, as a function of mass-to-charge ratio, $m/z$. In this way the example mass spectrum 390, $I(m/z)$ may be given as:

$$I(m/z) = c_1 I_1(m/z) + c_2 I_2(m/z) + c_3 I_3(m/z) + \eta(m/z)$$

Where $I_n$, $n = 1,2,3$, are the further mass spectrometry data 390-1; 390-2; 390-3, and $c_n$ are the scale factors for each further mass spectrometry data and $\eta(m\backslash z)$ is a term representing the measurement error and/or random noise in the mass spectrometer arrangement 101; 201 used to generate the example mass spectrum 390.

It will be appreciated that the above illustration of mass spectrometry data and the contributions for further mass spectrometry data are applicable to any of the mass spectrometry stages described previously. For example for an MS1 stage or experiment the example mass spectrometry data 390 comprises the intensities of ions of the molecular species in the sample 100; 200. Each of the further mass spectrometry data 390-1; 390-2; 390-3 is (or represents) an isotope pattern corresponding to a respective molecular species in the sample 100; 200. An isotope pattern for a molecular species comprises the relative intensities of the ions at each m/z ratio expected from a given sample of that molecular species. In other words an isotope pattern will be understood as the pattern of relative abundancies of a number of isotopes of a particular molecular species. As such, an isotope pattern may allow calculation of the relative abundance of one isotope of a particular molecular species with respect to another isotope of the particular molecular species. An isotope pattern for a molecular species may, therefore, comprise (or represent) a number of mass channels, each mass channel corresponding to a respective isotope of the molecular species. The isotope pattern further comprises a respective intensity (or abundance) for each mass channel of the isotope pattern.

An isotope pattern may be represented by (or may comprise) a set of intensity scale factors, $S_p$, relating the expected intensity at a given m/z ratio (or mass channel), $M_p$, for an overall concentration, $C$, of a given molecular species. In other words the intensity scale factors may obey the relation $M_p \propto S_p C$. In the case where the intensity scale factors are normalized so that they sum to unity the stronger relation $M_p = S_p C$ would be obeyed. Another common normalization for an isotope pattern is to scale the intensity scale factors such that $Sj = 1$, where $M_j$ is the main or reporter m/z ratio (or mass channel). It will be appreciated that there are numerous mathematically equivalent ways in which such intensity scale factors may be constructed depending upon the units used for the various quantities, and/or the normalization scheme used.

In this way it would be appreciated that, an overall concentration of a molecular species may be scaled by the isotope pattern to obtain expected mass spectrometry data comprising expected intensities for the mass channels of the isotopes of said molecular species. As such an isotope pattern itself may be considered to be a representation of a mass spectrum.

As a further example for an MS2 stage (or a higher MSn stage, where n>2) the example mass spectrometry data 390 comprises the intensities of fragment ions of the precursor molecular species in the sample 100; 200. Each of the sets of further mass spectrometry data 390-1; 390-2; 390-3 is (or represents) a fragment mass spectrum corresponding to (or derived from) a respective precursor molecular species in the sample 100; 200. As such a set of further mass spectrometry data 390-1; 390-2; 390-3 in this example comprises the intensities of each expected fragment ion from the fragmentation of a given precursor molecular species. It will be appreciated that the set of further mass spectrometry data may comprise the intensities of a sub-set of expected fragment ions from the fragmentation of a given precursor molecular species. As above, such a set of further mass spectrometry data may provide the relative abundancies of the fragment ions for a given precursor molecular species. A fragment mass spectrum for a precursor molecular species may, therefore, comprise (or represent) a number of mass channels, each mass channel corresponding to a respective fragment ion produced by fragmentation of the precursor molecular species. The fragment mass spectrum further comprises a respective intensity (or abundance) for each mass channel of the fragment ion mass spectrum.

It will be appreciated from the above discussion that for a given experimental mass spectrum produced by a MS1 stage and a given mass spectrum produced by a MS2 (or higher) stage an analogous situation may occur where the mass spectrum comprises contributions from more than one molecular species (in the MS1 case) which may be named precursor molecular species (in the MS2 or higher case). The resulting mass spectra may therefore be considered to comprise a linear combination of the isotope patterns of the molecular species (in the MS1 case) or the fragment mass spectra (in the MS2 or higher case). In both cases such resulting mass spectra may be considered to be chimeric mass spectra.

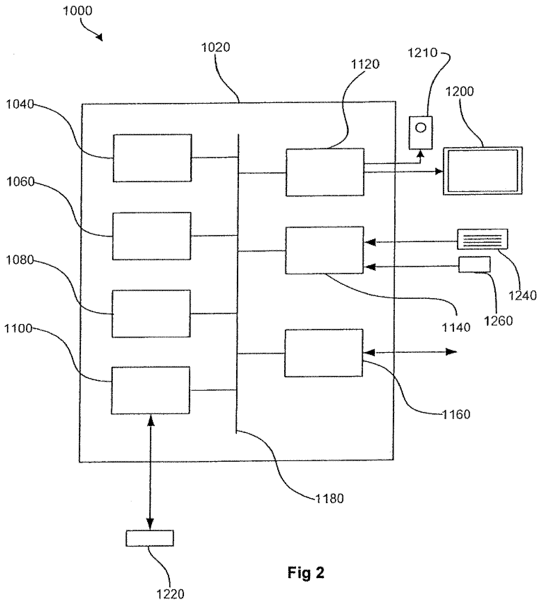

Figure 2 schematically illustrates an example of a computer system 1000 that may be used in embodiments of the invention. The system 1000 comprises a computer 1020. The computer 1020 comprises: a storage medium 1040, a memory 1060, a processor 1080, an interface 1100, a user output interface 1120, a user input interface 1140 and a network interface 1160, which are all linked together over one or more communication buses 1180.

The storage medium 1040 may be any form of non-volatile data storage device such as one or more of a hard disk drive, a magnetic disc, an optical disc, a ROM, etc. The storage medium 1040 may store an operating system for the processor 1080 to execute in order for the computer 1020 to function. The storage medium 1040 may also store one or more computer programs (or software or instructions or code).

The memory 1060 may be any random-access memory (storage unit or volatile storage medium) suitable for storing data and/or computer programs (or software or instructions or code).

The processor 1080 may be any data processing unit suitable for executing one or more computer programs (such as those stored on the storage medium 1040 and/or in the memory 1060), some of which may be computer programs according to embodiments of the invention or computer programs that, when executed by the processor 1080, cause the processor 1080 to carry out a method according to an embodiment of the invention and configure the system 1000 to be a system according to an embodiment of the invention. The processor 1080 may comprise a single data processing unit or multiple data processing units operating in parallel or in cooperation with each other. The processor 1080, in carrying out data processing operations for embodiments of the invention, may store data to and/or read data from the storage medium 1040 and/or the memory 1060. The processor 1080 may comprise one or more graphics processing units (GPUs) operating in cooperation with other data processing units of the processor 1080.

The interface 1100 may be any unit for providing an interface to a device 1220 external to, or removable from, the computer 1020. The device 1220 may be a data storage device, for example, one or more of an optical disc, a magnetic disc, a solid-state-storage device, etc. The device 1220 may have processing capabilities - for example, the device may be a smart card. The interface 1100 may therefore access data from, or provide data to, or interface with, the device 1220 in accordance with one or more commands that it receives from the processor 1080.

The user input interface 1140 is arranged to receive input from a user, or operator, of the system 1000. The user may provide this input via one or more input devices of the system 1000, such as a mouse (or other pointing device) 1260 and/or a keyboard 1240, that are connected to, or in communication with, the user input interface 1140. However, it will be appreciated that the user may provide input to the computer 1020 via one or more additional or alternative input devices (such as a touch screen). The computer 1020 may store the input received from the input devices via the user input interface 1140 in the memory 1060 for the processor 1080 to subsequently access and process, or may pass it straight to the processor 1080, so that the processor 1080 can respond to the user input accordingly.

The user output interface 1120 is arranged to provide a graphical/visual and/or audio output to a user, or operator, of the system 1000. As such, the processor 1080 may be arranged to instruct the user output interface 1120 to form an image/video signal representing a desired graphical output, and to provide this signal to a monitor (or screen or display unit) 1200 of the system 1000 that is connected to the user output interface 1120. Additionally or alternatively, the processor 1080 may be arranged to instruct the user output interface 1120 to form an audio signal representing a desired audio output, and to provide this signal to one or more speakers 1210 of the system 1000 that is connected to the user output interface 1120.

Finally, the network interface 1160 provides functionality for the computer 1020 to download data from and/or upload data to one or more data communication networks.

It will be appreciated that the architecture of the system 1000 illustrated in figure 2 and described above is merely exemplary and that other computer systems 1000 with different architectures (for example with fewer components than shown in figure 2 or with additional and/or alternative components than shown in figure 2) may be used in embodiments of the invention. As examples, the computer system 1000 could comprise one or more of: a personal computer; a server computer; a mobile telephone; a tablet; a laptop; a television set; a set top box; a games console; other mobile devices or consumer electronics devices; distributed (or cloud) computing systems etc.

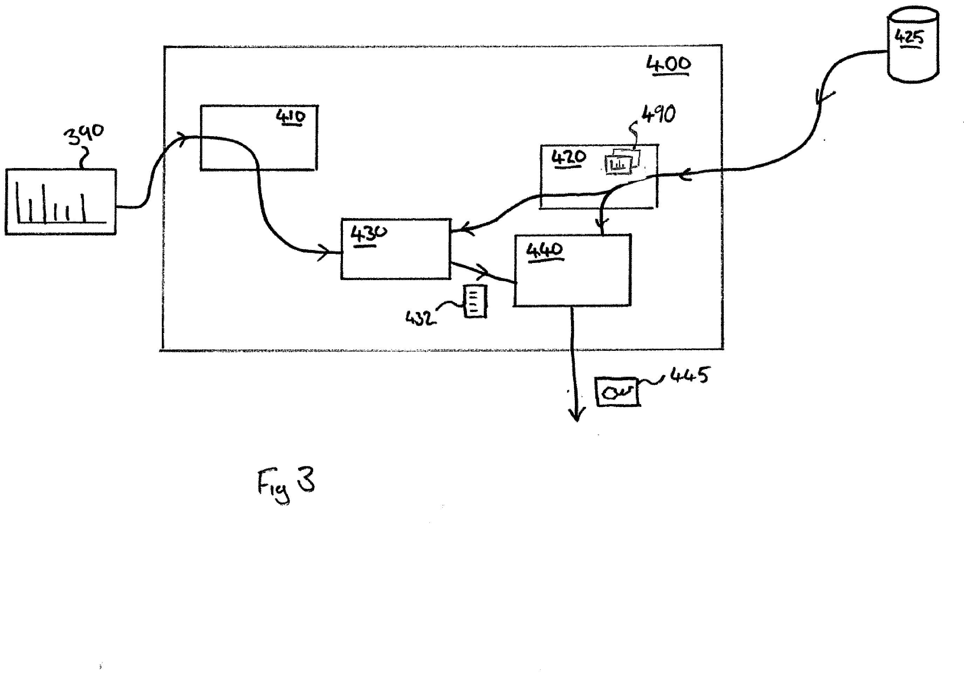

Figure 3 schematically illustrates a logical arrangement of an example analysis system 400 that may be used to analyze mass spectrometry data 390, such as the mass spectrometry data 190; 290; 390 discussed above in relation to figures 1a-1c. The analysis system 400 comprises a receiver module 410, a candidate selection module 420, an optimization module 430, and an indication module 440. The analysis system 400 may be implemented (or embodied) on one or more computer systems, such as the example computer system 1000 described above in relation to figure 2.

The receiver module 410 is arranged to receive mass spectrometry data 390. Typically, the receiver module 410 is arranged to receive the mass spectrometry data 390 from a mass spectrometer arrangement 101; 201 coupled to (or connected to) the analysis system 300. However, it will be appreciated that the receiver module 410 may be arranged to receive the mass spectrometry data 390 from any suitable source, including a data storage device, a cloud computing service, a test data generation program etc. The mass spectrometer data 390 may be received in the form of a mass spectrum as described previously. As set out previously, the mass spectrometry data 390 has a plurality of mass channels each having (or being filled with) a respective intensity (or intensity value).

The candidate selection module 420 is arranged to obtain a set 490 of candidate mass spectrometry data for the received mass spectrometry data 190. The set 490 of candidate mass spectrometry data may be in the form of a set of candidate mass spectra as discussed previously. For ease of understanding candidate mass spectra will be used in the following discussion but it will be appreciated that the following discussion could apply equally to candidate mass spectrometry data in other forms. Each candidate mass spectrum typically corresponds to a respective candidate molecular species. The candidate mass spectra may be obtained from a mass spectra database 425. Additionally, or alternatively it will be appreciated that some or all of the candidate mass spectra may be generated on demand (or on the fly). It will be appreciated that in some embodiments the candidate selection module 420 may select all candidate mass spectrometry data available in the mass spectra database 425 as the set of candidate mass spectrometry data.

The candidate mass spectra may comprise mass spectra generated by mass spectrometry experiments on corresponding molecular species. Preferably the mass spectrometry experiments have samples which consist of pure (or substantially pure) samples of the corresponding molecular species. Additionally, or alternatively the candidate mass spectra comprise theoretical (or predicted) mass spectra. A theoretical (or calculated or predicted) mass spectrum is typically a mass spectrum generated based on a theoretical model of a mass spectrometry experiment. In particular such models may be generated using machine learning based on suitable training data. An example of the generation of theoretical mass spectra is the INFERYS system by MSAID GmbH. Other examples include those described in Gessulat, S., Schmidt, T., Zolg, D.P. et al. Prosit: proteome-wide prediction of peptide tandem mass spectra by deep learning. Nat Methods 16, 509-518 (2019). DOI: 10.1038/s41592-019-0426-7, "pDeep: Predicting MS/MS Spectra of Peptides with Deep Learning" Zhou et al. Analytical Chemistry 2017 89 (23), 12690-12697 DOI: 10.1021/acs.analchem.7b02566 and "MS2PIP: a tool for MS/MS peak intensity prediction", Sven Degroeve, Lennart Martens, Bioinformatics, Volume 29, Issue 24, 15 December 2013, Pages 3199-3203, DOI: 10.1093/bioinformatics/btt544. Examples of methods for generating theoretical (or calculated) isotope patterns include "Poisson Model To Generate Isotope Distribution for Biomolecules" Rovshan G. Sadygov, Journal of Proteome Research 2018 17 (1), 751-758 DOI: 10.1021/acs.jproteome.7b00807; "BRAIN: A Universal Tool for High-Throughput Calculations of the Isotopic Distribution for Mass Spectrometry" Dittwald et al., Analytical Chemistry 2013 85 (4), 1991-1994 DOI: 10.1021/ac303439m; "BRAIN 2.0: Time and Memory Complexity Improvements in the Algorithm for Calculating the Isotope Distribution", Dittwald, P. et al., J. Am. Soc. Mass Spectrom. 25, 588-594 (2014) DOI: https://doi.org/10.1007/s13361-013-0796-5; "Accelerated Isotope Fine Structure Calculation Using Pruned Transition Trees", Martin Loos et al., Analytical Chemistry 2015 87(11),5738-5744 DOI: 10.1021/acs.analchem.5b00941; "IsoSpec: Hyperfast Fine Structure Calculator", Mateusz K. Łącki et al., Analytical Chemistry 2017 89(6),3272-3277 DOI: 10.1021/acs.analchem.6b01459; "IsoSpec2: Ultrafast Fine Structure Calculator", Mateusz K. Łącki et al., Analytical Chemistry 2020 92(14),9472-9475 DOI: 10.1021/acs.analchem.0c00959; and "Fast Exact Computation of the k Most Abundant Isotope Peaks with Layer-Ordered Heaps", Patrick Kreitzberg et al., Analytical Chemistry 2020 92(15),10613-10619 DOI: 10.1021/acs.analchem.0c01670. It will be appreciated that the use of theoretical mass spectra as the candidate mass spectra in the presently described invention is particularly advantageous as a theoretical mass spectrum corresponding to a particular molecular species can be considered to be pure. In other words the theoretical mass spectrum comprises only contributions from the corresponding molecular species. Conversely an experimental mass spectrum, such as those obtained from experimental mass spectrum libraries, may include contributions from other molecular species that may have contaminated the initial sample used to generate the experimental mass spectrum or may contain experimental artifacts, noise etc. As will become apparent form the discussions below such contamination in the candidate mass spectra may increase errors in the subsequent identification of the received mass spectrometry data 390.

The candidate selection module 420 may be arranged to select one or more candidate mass spectra as part of the set 490 of candidate mass spectra based on the received mass spectrometry data 390. For example the received mass spectrometry data 390 may correspond to (or have been generated using) a given isolation window. In other words the sample 100; 200 used in the mass spectrometer arrangement to generate the mass spectrometry data may have undergone selection so that only ions within a given mass-to-charge range were analyzed (or detected). In such scenarios the candidate selection module 420 may be arranged to select candidate mass spectra corresponding to candidate molecular species that are within the given isolation window (or have a mass-to-charge ratio inside mass-to-charge ratio range). The candidate selection module 420 may further be arranged to discard (or not select) candidate mass spectra corresponding to candidate molecular species that are not within the given isolation window (or have a mass-to-charge ratio outside the mass-to-charge ratio range). Additionally, or alternatively the candidate selection module 420 may be arranged to select candidate mass spectra based on a similarity with the received mass spectrometry data 390. In particular, candidate mass spectra may be selected based on a pre-defined threshold of peaks in the candidate mass spectra being present in the received mass spectrometry data 390. For example, the candidate selection module 420 may be arranged to discard (or not select) a mass spectrum as a candidate mass spectrum if the mass spectrum has fewer than a threshold number of peaks present in mass channels that have peaks present in the received mass spectrometry data 390. This may be thought of as selecting candidate mass spectra based on a peak presence score.

Additionally, or alternatively the candidate selection module 420 may be arranged to select candidate mass spectra based on any one or more of the following criteria:

- physicochemical properties of the candidate molecular species;
- a predetermined likelihood that a given candidate molecular species is detectable in a mass spectrometer;
- the isolation window of the received mass spectrometry data 390;
- a retention time corresponding to the received mass spectrometry data 390;
- charge of the precursor molecular species;
- presence and/or absence of peaks in the received mass spectrometry data 390 compared to a given candidate molecular species;
- number of shared peaks between the received mass spectrometry data 390 and the candidate molecular species (for example the minimum number may be 2 or 3 or more);
- similarity and/or dissimilarity criteria comparing the intensities of candidate spectra to the intensities in the received mass spectrometry data 390;
- a normalized spectral contrast angle (i.e. cosine similarity); a Pearson correlation; a Spearman correlation; and so on.

The candidate selection module may be arranged to to select candidate mass spectra based on mathematical combinations (e.g. multiplication, division, etc.) of intensity-based similarity/dissimilarity criteria (e.g. comparing the intensities of candidate spectra to the intensities in the received mass spectrometry data 390, cosine similarity, Pearson correlation, spearman correlation, and so on) with peak-presence/absence-based similarity/dissimilarity criteria (e.g. number of shared peaks between the received mass spectrometry data 390 and the candidate molecular species).

Additionally, or alternatively the candidate selection module 420 may weigh matches between the peaks present in a mass spectrum in mass channels that have peaks present in the received mass spectrometry data 390. Such weighting may be based on, for example, the intensity of the peak in the received mass spectrometry data 390. In this way matches for the most abundant peaks in the received mass spectrometry data 390 are given a greater weighting. This may be thought of as selecting candidate mass spectra based on a peak intensity score. It will be appreciated that combining intensity-based similarity/dissimilarity criteria with peak-presence/absence-based similarity/dissimilarity criteria may alleviate weaknesses that either class of criteria has when used in isolation.

The candidate selection module 420 may also be arranged to score candidate mass spectra against the received mass spectrometry data 290 based on based on machine learning algorithms, for example Linear Discriminant Analysis (LDA). In particular the candidate selection module 420 may be arranged to select (or discard) mass spectra as candidate mass spectra based on a LDA score. Such selection may comprise selecting the mass spectra having a score above a predetermined threshold. Alternatively, such selection may comprise ranking the mass spectra by said LDA score and selecting a predetermined number of mass spectra having the highest scores as the candidate mass spectra.

Additionally, or alternatively, the candidate selection module may be arranged to select candidate mass spectra based on information obtained from a further set of received mass spectrometry data. For example the received mass spectrometry data 390 may correspond (or have been generated using) no fragmentation at all, while the further received mass spectrometry data may correspond (or have been generated using) fragmentation. In other words the mass spectrometry data 390 may correspond to MS1 spectra and the further set of received mass spectrometry data may correspond to MS2 spectra. It will be appreciated that the two sets of received mass spectrometry data may correspond to (or have been generated using) the same (or similar) separation parameter value. In such scenarios the candidate selection module 420 may be arranged to select candidate mass spectra corresponding to candidate molecular species for the received mass spectrometry data 390 that are within a given window of the separation parameter. The skilled in the art will appreciate that classical peak shapes (e.g. Rayleigh distribution, Gaussian distribution) describing the separation of candidate molecular species can be taken into account for the determination of this window.

The optimization module 430 is arranged to optimize a set of mass spectra coefficients 432 for the set of candidate mass spectra 490, based on the received mass spectrum. The set 432 of mass spectra coefficients may be thought of as defining (or specifying) a linear combination of the candidate mass spectra. Each candidate mass spectrum may be assigned (or correspond to) a mass spectra coefficient. The optimization module 430 is usually arranged to require (or enforce) these coefficients to be non-negative. The mass spectra coefficient for a candidate mass spectrum scales the intensity of each peak in the candidate mass spectrum by the value of the mass spectrum coefficient. For example, take P candidate mass spectra each comprising a set of intensities $I_{p,m}$, where $p = 1, 2, ..., P$ is an index over the mass spectra and m is an index over the mass channels for each candidate mass spectrum. The set 432 of mass spectra coefficients $\beta_p$ define a further mass spectrum being a linear combination of the candidate mass spectra scaled by the corresponding mass spectra coefficients. This further mass spectrum has a set of intensities:

$$I_m = \sum_{p=1}^{P} \beta_p I_{p,m}$$

It will be appreciated that there may also be a further coefficient $\beta_0$ which may represent a constant intensity offset for the further mass spectrum. In other words $I_m = \beta_0 + \sum_{p=1}^{P} \beta_p I_{p,m}.$ For ease of understanding this is omitted from the discussion below. However, it will be appreciated that this may be included should a constant offset be desired.

In order to optimize the set of mass spectra coefficients the optimization module 430 is typically arranged to vary the respective values of the mass spectra coefficients (or mass spectra coefficient values) based on an objective function.

The objective function relates (or comprises a term relating) the linear combination of the candidate mass spectra, as defined by (or according to) the set of mass spectra coefficients, to the received mass spectrum 390. Typically, the objective function provides a measure of difference between the linear combination of mass spectra and the received mass spectrum 390. The optimization module may be arranged to vary the respective values of the mass spectra coefficients with the aim of obtaining an extremum value of the objective function. In particular, where the objective function provides a measure of difference between the linear combination of mass spectra and the received mass spectrum 390 the values of the mass spectra coefficients may be varied with the aim of minimizing said objective function. It will be appreciated however that such minimization problems may be recast as a maximization, or as obtain a saddle point of an objective function by suitable re-casting of the objective function. It will be appreciated form the discussion below that as numerical optimization methods may be used to obtain the coefficients $\beta_p$ it may be possible to include substantially negligible terms that are non-linear (such as polynomial and the like) in intensity in the objective function whilst having no appreciable effect on the values of the coefficients obtained. Such an objective function may still be thought of as relating the linear combination of the candidate mass spectra, as defined by (or according to) the set of mass spectra coefficients, to the received mass spectrum 390.

In other words, the optimization module 430 may be thought of as being arranged to obtain a set of values of the mass spectra coefficients, $\beta_p$, such that for each mass channel m:

$$I_m^{\exp} = \sum_{p=1}^{P} \beta_p I_{p,m} + \epsilon_m$$

where $I_m^{\exp}$ is the intensity values of the received mass spectrum for the mass channel m and $\varepsilon_m$ is an error term. The aim of the optimization may be thought of as obtaining a set 432 of coefficients $\{\beta_p\}$ so that the set of error terms $\{\varepsilon_m\}$ (or a function thereof) are substantially minimized. In this way it will be understood that the optimization may be a linear regression optimization process. In particular the sum of the square of the error terms may be minimized (or sought to be minimized) by the optimization.

As such the objective function may comprise a term representing the sum of the squares of the respective difference between the intensities of the received mass spectrum 390 and the respective corresponding intensities of each candidate mass spectrum scaled by the coefficient of the candidate mass spectrum. Mathematically this term may be cast as:

$$\sum_m^{M} \left(I_m^{\exp} - I_m^T \beta\right)^2$$

where $M$ is the number of mass channels, $I_m = (I_{1,m}, I_{2,m}, I_{3,m}, ..., I_{P,m})$ a vector of the intensities of each candidate mass spectrum of the set of candidate mass spectra at the mass channel $m$, and $\beta = \beta_1, \beta_2, \beta_3, ..., \beta_P)$ a vector of the mass spectra coefficients. It will be appreciated that in some embodiments the square root of the above term may be used instead.

It will be appreciated that other treatments of the error term may be used in the minimization in place of the sum of the square of the error terms set out above.

For example the mean absolute error may be minimized. As such the objective function may comprise a term representing the sum of the absolute respective differences between the intensities of the received mass spectrum 390 and the respective corresponding intensities of each candidate mass spectrum scaled by the coefficient of the candidate mass spectrum. Mathematically this term may be cast as:

$$\sum_{m}^{M} |I_m^{\exp} - \boldsymbol{I}_m^T \boldsymbol{\beta}|$$

It will be appreciated that other treatments of the error term may, in effect, result in the term above taking the form of one or more existing loss functions. Examples of such loss functions include: Huber loss (or smooth mean absolute error); Cosine similarity (or spectral angle); LogCosh loss; Maximum likelihood derived loss functions (MLE); Cross-entropy; Hinge loss; LINEX loss; and so on.

The optimization module 430 is also arranged to perform said varying subject to a regularization (or constraint). The regularization is typically enforced by the inclusion of a regularization (or penalty) term in the objective function. The regularization is arranged to constrain (or reduce or otherwise penalize the increase of) the number of non-zero mass spectra coefficients. The regularization term may be arranged to move the objective function away from an extremum value in dependence on increasing the number of nonzero coefficients. In this way the regularization term may be directly dependent on the number of non-zero coefficients. As such, it will be appreciated that the regularization term may be arranged to enforce sparsity in the vector of coefficients. Additionally, or alternatively the regularization term may be indirectly dependent on the number of non-zero coefficients. In particular the regularization term may be proportional to the sum of the magnitude of the coefficients.

For example LASSO (or least absolute shrinkage and selection operator) regularization may be used. In this case the regularization term may take the form:

$$\lambda \|\boldsymbol{\beta}\|_1 = \lambda \sum_{p}^{P} |\beta_p|$$

where $\lambda$ may be varied to vary the strength of the regularization, and therefore the strength of constraint on the number of non-zero coefficients.

In the case of LASSO regularization the objective function may take the form (or comprise the terms):

$$\sum_{m}^{M} \left(I_m^{\exp} - \boldsymbol{I}_m^T \boldsymbol{\beta}\right)^2 + \lambda \|\boldsymbol{\beta}\|_1$$

In such an example the optimizing may be thought of as comprising solving a minimization problem:

$$\min \left\{ \sum_{m}^{M} \left(I_m^{\exp} - \boldsymbol{I}_m^T \boldsymbol{\beta}\right)^2 + \lambda \|\boldsymbol{\beta}\|_1 \right\}$$

subject to the coefficients $\beta_p$ having non-negative values. Such a minimization problem is known as non-negative L1-regularized regression.

It will be appreciated that further regularization terms may also be included in the objective function in addition to any of those set out above. In particular an $L_2$-regularization term may be used. An example of such a term would be the regularization term used in so-called "ridge regression". When combined with LASSO regularization as discussed above this can be termed "elastic net" regression. Here the objective function may take the form (or comprise the terms):

$$\sum_{m}^{M} \left(I_m^{\exp} - \boldsymbol{I}_m^T \boldsymbol{\beta}\right)^2 + \lambda \alpha \|\boldsymbol{\beta}\|_1 + \lambda \frac{(1 - \alpha)}{2} \boldsymbol{\beta}^2$$

where $\alpha$ is a parameter between 0 and 1 which controls the relative strengths of the two regularization terms. Suitable

values for $\alpha$ may be determined by experimentation and these are chosen to ensure that the LASSO regularization term enforces sparsity in the vector of coefficients.

The strength of the regularization (such as the value of $\lambda$) may be predetermined, for example based on previous testing to obtain a desired number of non-zero coefficients. Additionally, or alternatively the optimization module 430 may be arranged to carry out multiple optimizations whilst varying the strength of the regularization. In this way the number of non-zero coefficients may be minimized with respect to a measure of quality of the coefficients. For example the number of non-zero coefficients may be minimized with respect to an absolute difference of the intensities of the linear combination of mass spectra according to the mass spectra coefficients and the received mass spectra not exceeding a predetermined threshold value. It will also be appreciated that other model selection criteria or methods may additionally, or alternatively, be used to select $\lambda$. Examples of these include any of: Akaike information criterion (AIC);

Corrected Akaike information criterion (AICc); Bayesian information criterion (BIC); Adjusted R-Squared (R2adj); Cross-validation (CV); Stepwise regression; and so on.

It will be appreciated that the optimization module 430 may be arranged to implement the varying of the mass spectra coefficients based on the objective function using a numerical optimization technique of which many examples are known in the art. In particular the varying may be implemented using (or comprise or be based on) an iterative method (or procedure). As such, the optimization of the mass spectra coefficients described above may not actually obtain an extremum value of the objective function. The optimization described above may be complete (or successful or may terminate) when a value of the objective function is obtained that is suitably close (or estimated to be suitably close) to an extremum value (or estimated or predicted extremum value) of the objective function. If the varying is implemented using an iterative method the optimization described above may be complete if any of the following conditions are met:

(a) a predefined number of iterations is exceeded or met;
(b) the change in the value of an objective function with respect to a previous iteration is below a predefined threshold;
(c) the change in value (or values) of one or more mass spectra coefficients with respect to a previous iteration is below a predefined threshold;
(d) a predefined amount of time has elapsed;
(e) a predefined number of processor cycles have elapsed;
(f) a predefined maximum number of non-zero coefficients is exceeded (or met);
(g) a predefined maximum fraction of explained deviance is exceeded (or met);
(h) the value of the objective function without the regularization term is within a predefined threshold of its extremum value; etc.

Typically the varying is implemented using coordinate descent. However, the varying may be implemented, in whole or in part, using any of: a finite differences method e.g. such as Newton's method; a Quasi-Newton method; a conjugate gradient method; a steepest descent method; proximal minimization; subgradient methods; proximal gradient methods; least angle regression (LARS); quadratic programming; convex programming etc.

The optimization module 430 is arranged to provide the set of optimized mass spectra coefficients 432 to the indication module 440.

The indication module 440 is arranged to provide, for one or more of the candidate molecular species, a respective indication 445 of a match in the received mass spectrum 390, based at least in part on the optimized set of mass spectra coefficients 432. Typically, an indication 445 is provided to a user, for example via user output interface 1120. Additionally or alternatively the indication 445 may be stored and/or provided to a further system for additional processing.

It will be understood that a non-zero mass spectrum coefficient in the set of optimized mass spectra coefficients 445 may be considered to indicate the presence of the corresponding candidate mass spectrum in the received mass spectrum 390. Consequently a non-zero mass spectrum coefficient may be considered to indicate the presence of the corresponding candidate molecular species in the received mass spectrometry data 390 (or the sample corresponding to the received mass spectrometry data 390). It will also be appreciated that the magnitude of a mass spectrum coefficient in the set of optimized mass spectra coefficients 445 may be considered proportional to the abundance (or relative abundance) of the corresponding candidate molecular species in the received mass spectrometry data 390.

The respective indication may comprise any one or more of: a flag (or other marker) indicating (or signaling) the presence of the candidate molecular species in the received mass spectrometry data 390; a relative (or absolute) abundance of the candidate molecular species in the received mass spectrometry data 390; the respective optimized mass spectrum coefficient; etc.

The indication module 440 may be arranged to indicate the presence of a molecular species if the corresponding mass spectrum coefficient exceeds a predetermined value. In this way the most likely (or most abundant) molecular species may be indicated (or identified). Additionally, or alternatively the indication module 440 may be arranged to indicate the presence of only a predetermined number of molecular species, for example those with the largest mass spectra coefficients (or largest contributions to the received mass spectrum 390).

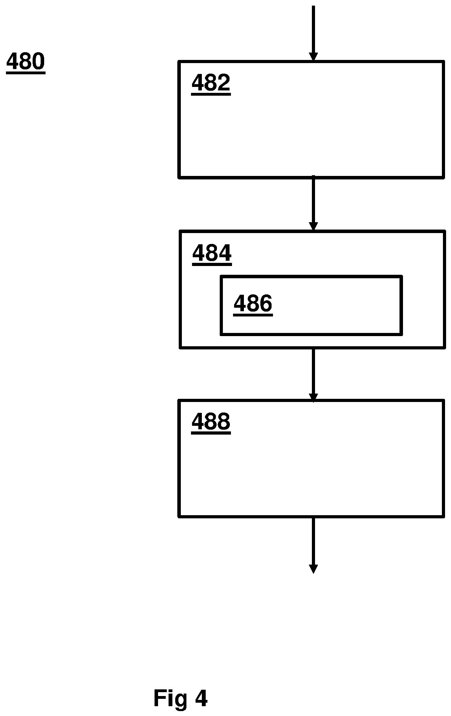

Figure 4 is a flow diagram schematically illustrating an example method 480 of identifying one or more molecular species represented in received mass spectrometry data 390, such as may be carried out by the system 400 of figure 3.

At a step 482, which may be carried out by the candidate selection module 420, a set 490 of candidate mass spectra for the received mass spectrometry data 390 is obtained. Each candidate mass spectrum corresponds to a respective candidate molecular species. One or more candidate mass spectra are typically selected as part of the set 490 of candidate mass spectra based on the received mass spectrometry data 390. In particular, the candidate mass spectra may be selected based on similarity (or one or more similarity scores) with the received mass spectrometry data as described above. For received mass spectrometry data 390 generated from an MS2 (or MSn, where n> 2) experiment the received mass spectrometry data 390 may correspond to (or have been generated using) a given isolation window. In this case the step 482 may comprise selecting candidate mass spectra for molecular species corresponding to m/z values that fall within the isolation window.

At a step 484 a set 432 of mass spectra coefficients for the set 490 of candidate mass spectra are optimized, based on the mass spectrum. The step 484 may be carried out by the optimization module 430.

The step 484 comprises a step 486. At the step 486 the mass spectra coefficient values are varied based on an objective function. As described above the objective function relates a linear combination of the candidate mass spectra, according to the mass spectra coefficients, to the received mass spectrometry data 390. The step 486 is performed subject to a regularization term of the objective function constraining the number of non-zero mass spectra coefficient values. As described previously the regularization term may be proportional to the sum of the magnitude of the coefficients.

For example LASSO (or least absolute shrinkage and selection operator) regularization may be used. In such an example the step 484 of optimizing may be thought of as comprising solving a minimization problem:

$$\min\left\{\sum_{m}^{M}\left(I_m^{\exp} - \boldsymbol{I}_m^T\boldsymbol{\beta}\right)^2 + \lambda\|\boldsymbol{\beta}\|_1\right\}$$

subject to the coefficients $\beta_p$ having non-negative values.

At a step 488, for one or more of the candidate molecular species, a respective indication of a match in the mass spectrometry data 390 is provided, based at least in part on the optimized set 432 of mass spectra coefficients. The respective indication may comprise any one or more of: a flag (or other marker) indicating (or signaling) the presence of the candidate molecular species in the received mass spectrometry data 390; a relative (or absolute) abundance of the candidate molecular species in the received mass spectrometry data 390; the respective optimized mass spectrum coefficient; etc.

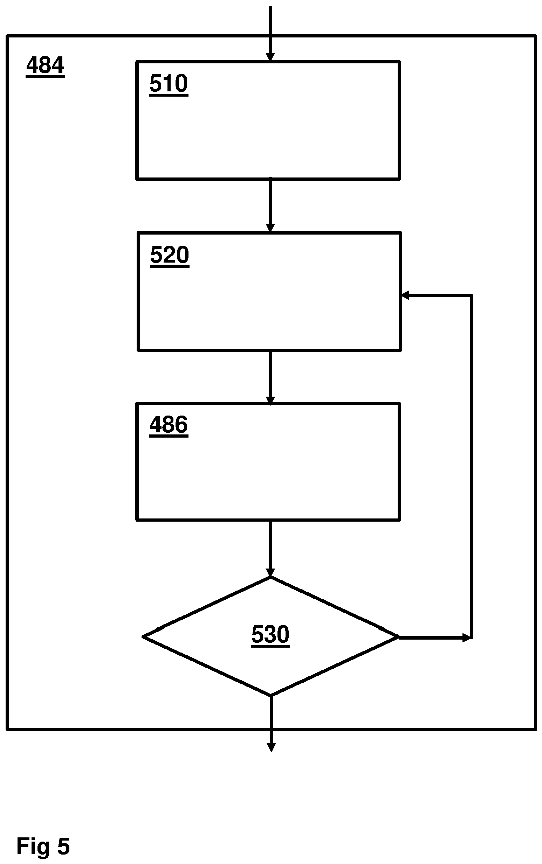

Figure 5 is a flow diagram schematically illustrating a further example of the step of optimizing such as the step 484 of optimizing of the method 480 shown in figure 4.

In the further example the regularization term comprises a parameter specifying the degree of regularization, such as the parameter $\lambda$ described above in relation to the LASSO regularization term.

At a step 510 an initial value for the degree of regularization is obtained. Typically the initial value is selected as an extremum value determined based upon the regularization term used. In particular, degree of regularization may be chosen such that the coefficients are analytically zero. It will be appreciated that when for any change in the coefficients away from zero the change in the regularization term is strictly larger and of opposite sign than the change in all other terms of the objective function then the coefficients can be considered analytically zero. For LASSO regularization this may be achieved when $\lambda$ is suitably large. However it will be appreciated that any initial value for the degree of regularization may be selected though sub-optimal initial values may lead to increased computational effort to reach the convergence discussed below.

At a step 520 the degree of regularization is reduced by a predetermined step value. It will be appreciated that the size of the step value may be selected based on a desired speed of convergence.

At the step 486 the mass spectra coefficient values are varied based on the objective function using the current degree of regularization as described above. This step 486 of varying results in a set of optimized mass spectra coefficients being generated for the current degree of regularization.

The step 520 and the step 486 are then iterated until convergence criteria are met (or satisfied). In this way the degree of regularization is varied until a convergence of the optimized set of mass spectra coefficients is obtained (or other stopping criteria is met).

At the step 530 one or more convergence criteria are tested. The convergence (or stopping) criteria may additionally, or alternatively comprise any one or more of: a predefined number of iterations being exceeded or met; the change in value (or values) of one or more mass spectra coefficients with respect to a previous iteration being below a predefined threshold; a predefined amount of time having elapsed; a predefined number of processor cycles having elapsed; a minimum number of non-zero coefficients being found etc.

It will be appreciated that the iteration process may be accelerated by using the values of the mass spectra coefficients obtained in the previous iteration as starting values for the mass spectra coefficients in the next step 486 of varying.

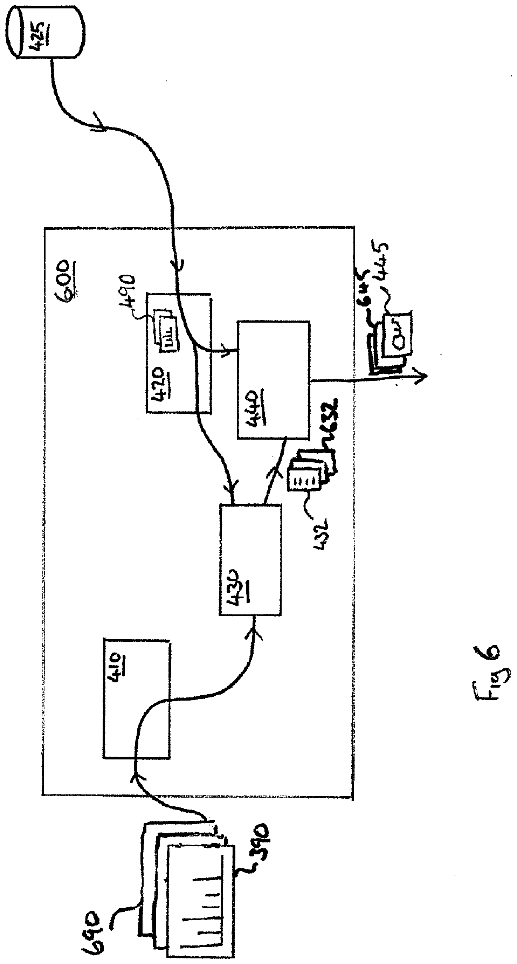

Figure 6 schematically illustrates an exemplary analysis system 600 according to one embodiment of the invention. The system 600 is the same as the system 400 of figure 3, except as described below. Therefore, features in common to the system 600 and the system 400 have the same reference numerals and shall not be described again.

The receiver module 410 is arranged to receive a plurality 690 of items of mass spectrometry data 390 (or a plurality of mass spectra). Each received mass spectrum 390 is a mass spectrum generated by a mass spectrum arrangement 101; 201 from a respective sample eluted at a respective value of a separation parameter from a separation device coupled to the mass spectrometer arrangement 101; 201 as described previously above. For example, the plurality of mass spectra may correspond to a chromatography experiment having as input a peptide mixture (such as that obtained by digestion of one or more proteins). In this example the peptides may elute from the chromatography device as a function of a separation parameter (such as retention time or a solvent gradient). Here each mass spectrum would correspond to the peptides eluted at a particular value of retention time or solvent gradient.

The optimization module 430 and the candidate selection module 420 are arranged substantially as described previously with the exception that they are arranged to act on each item of mass spectrometry data 690 in the same way they act upon the mass spectrometry data 390 described in figure 4. In this way it will be appreciated that the optimization module may be arranged to generate a plurality 632 of sets of optimized mass spectra coefficients 432, each corresponding to a respective item of mass spectrometry data 390. Similarly, the identification module 440 may be arranged to a set 645 of indications 445 corresponding to respective items of mass spectrometry data 390.

In addition, the optimization module may be arranged to further constrain values of the mass spectra coefficients for a received mass spectrum 390 at one value of the separation value based on the optimized values of mass spectrum coefficients for a received mass spectrum 390 at the preceding (and/or subsequent) value of separation parameter. In one example each coefficient may be penalized based on additional information or criteria.

In an example, a penalty term may be included that is proportional to the difference between the coefficient $\beta p_{,i}$ of a given candidate mass spectrum $p$ at one point $i$ in the separation parameter sequence and the coefficient $\beta_{p,j}$ of the same candidate mass spectrum $p$ at adjacent (and/or nearby points) points (such as $j = i - 1$, or $j = i + 1$) in the separation parameter sequence. Here the index $i = 1,2,3,...$ enumerates the sequence of received mass spectra 390 in increasing value of the separation parameter. It will be appreciated that when combined with the LASSO approach described previously the objective function may take the form:

$$\sum_{m}^{M}\left(I_{i,m}^{\exp} - I_{m}^{T}\boldsymbol{\beta}_{i}\right)^{2} + \lambda_{1}\|\boldsymbol{\beta}_{i}\|_{1} + \lambda_{2}\sum_{p}^{P}|\beta_{p,i} - \beta_{p,i-1}|$$

where $I_m = (I_{1,m}, I_{2,m}, I_{3,m}, ..., I_{P,m})$ is a vector of the intensities of each candidate mass spectrum at the mass channel m, $I_{i,m}^{\exp}$ the intensity at the mass channel $m$ of the $i^{th}$ received mass spectrum, $\beta_i = (\beta_{1,i}, \beta_{2,i}, \beta_{3,i}, ..., \beta_{P,i})$ a vector of the mass spectra coefficients for the $i^{th}$ received mass spectrum, $\lambda_1$ is the strength of regularization (previously denoted as $\lambda$), and $\lambda_2$ is the strength of the additional penalty term. It will be understood that this may be considered equivalent to a fused LASSO regularization problem. Whilst the above term has been described in relation to a previous point, $j = i - 1$, it will be appreciated that this discussion applies analogously to the next point, $j = i + 1$.

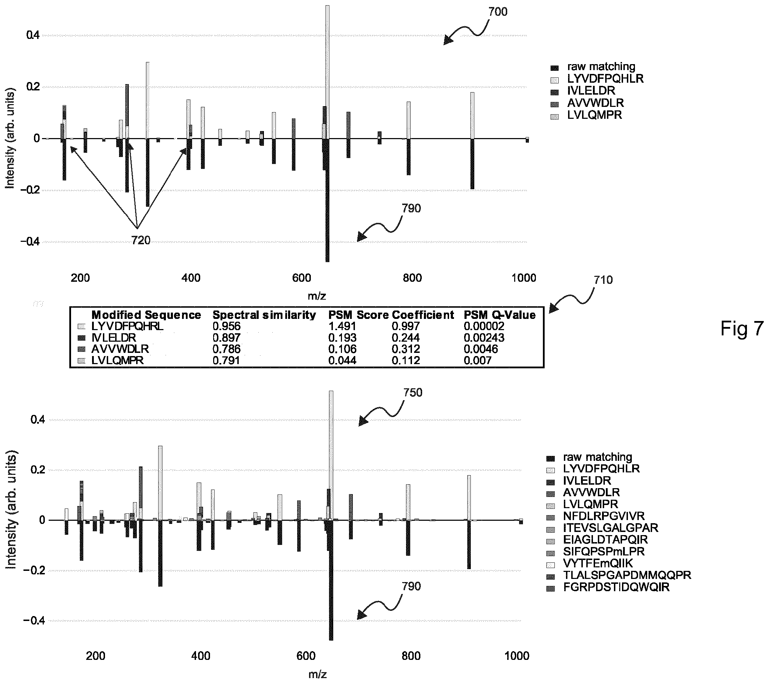

Figure 7 shows an example experimental mass spectrum 790 along with the linear combination of candidate mass spectra as calculated by an embodiment of the invention.

The intensity peaks shown below the x-axis on the graph 700 represent the experimental mass spectrum 790. The experimental mass spectrum 790 is generated by a MS2 mass spectrometer arrangement supplied with a sample of digested human proteins from cells grown in cell culture. The experimental mass spectrum 790 was acquired for mass 429.8997 with tolerance (isolation width) of 0.65 Da at retention time 48.96 min. Molecular species were fragmented with the normalized collision energy of 28 using higher-energy C-trap dissociation (HCD). The experimental mass spectrum was obtained using an Orbitrap ™ mass analyser. The spectrum shown in Fig 6 only contains experimental peaks with match to peaks from the predicted spectra (with a certain tolerance). In order to make the comparison clearer the intensity peaks corresponding to the LYVDFPQHLR $a_2$ ion, the Phenylalanine immonium ion, and the Tyrosine immonium ion known to be present in the sample, but not considered in the candidate mass spectra are omitted. A set of 29 candidate mass spectra including mass spectra corresponding to the peptides listed in Table 1 was selected and a set of mass spectra coefficients generated, in line with the systems and methods of the invention described previously.

*Table 1: Candidate peptides are denoted in single-letter amino acid code, lower case "m" denotes an oxidized methionine residue.*

| Unmodified Sequence | Modified Sequence | Precursor Charge | Molecular Weight |
|---|---|---|---|
| AKAIAINTFLPK | AKAIAINTFLPK | 3 | 1285.775808 |
| AVIGDASETALLK | AVIGDASETALLK | 3 | 1286.708182 |
| AVVWDLR | AVVWDLR | 2 | 857.4759375 |
| DLENWEKDIK | DLENWEKDIK | 3 | 1288.629932 |
| EIAGLDTAPQIR | EIAGLDTAPQIR | 3 | 1282.688115 |
| EPQVYVLDPPK | EPQVYVLDPPK | 3 | 1283.67612 |
| ITEVSLGALGPAR | ITEVSLGALGPAR | 3 | 1282.724501 |
| IVLELDR | IVLELDR | 2 | 856.5018179 |
| IYSNAGEQSFDKLLK | IYSNAGEQSFDKLLK | 4 | 1711.878101 |
| LASCPDSWVPR | LASCPDSWVPR | 3 | 1286.607757 |
| LVLQMPR | LVLQMPR | 2 | 855.5000438 |
| LYVDFPQHLR | LYVDFPQHLR | 3 | 1286.677157 |
| NFDLRPGVIVR | NFDLRPGVIVR | 3 | 1284.730255 |
| NFLTEMELDR | NFLTEmELDR | 3 | 1282.586353 |
| NVLDELR | NVLDELR | 2 | 857.4606814 |
| PAHSFLGK | PAHSFLGK | 2 | 855.4602874 |
| QLEQALQNNTQWLK | QLEQALQNNTQWLK | 4 | 1712.884583 |
| SLATTRPTVNADDLLK | SLATTRPTVNADDLLK | 4 | 1713.926114 |
| TAVFGFETSEAK | TAVFGFETSEAK | 3 | 1285.619033 |
| TLALSPGAPDMMQQPR | TLALSPGAPDMMQQPR | 4 | 1711.838561 |
| TLLQQNQQLKLDLR | TLLQQNQQLKLDLR | 4 | 1709.978818 |
| VYTFEMQIIK | VYTFEmQIIK | 3 | 1286.658061 |
| ECSSAILAIPPK | ECSSAILAIPPK | 3 | 1284.674774 |
| FGRPDSTIDQWQIR | FGRPDSTIDQWQIR | 4 | 1717.853618 |
| GCPDHHVVEIEWLK | GCPDHHVVEIEWLK | 4 | 1717.824626 |
| GLQLTQVHNQNLLLK | GLQLTQVHNQNLLLK | 4 | 1717.983903 |
| IAPPGPPDIPDAK | IAPPGPPDIPDAK | 3 | 1286.687053 |
| IVNEGDPIWIK | IVNEGDPIWIK | 3 | 1282.692138 |
| SIFQPSPMLPR | SIFQPSPmLPR | 3 | 1287.664543 |

11 non-zero coefficients were present in the set of optimized mass spectra coefficients of which the four largest are listed in the table 710 under the heading "coefficient". The intensity peaks shown above the x axis correspond to the linear combination of the candidate mass spectra scaled with their respective coefficients for the four largest mass spectra coefficients. The intensities of the combined intensity of the peaks well resembles the intensity of the matched experimental peaks. It may be noted that the peaks 720 of the linear combination involve contributions from multiple candidate mass spectra and again reproduce the corresponding peaks in the experimental mass spectrum closely.

A further graph 750 is also present in figure 7. The graph 750 is identical to the graph 700 with the exception that the linear combination of candidate mass spectra comprises all 11 candidate mass spectra having non-zero mass spectrum coefficients.

It will be appreciated that the mass spectra coefficients calculated with the above described systems and methods may be used in any number of ways to facilitate the analysis of chimeric mass spectra further to identifying and quantifying molecular species present in the mass spectrum. For example the mass spectra coefficients may enable the calculation of improved spectra similarity scores. It will be appreciated that a spectral similarity score is often used to gauge how well two mass spectra match.

Spectral similarity scores are calculated between an experimental spectrum and theoretical mass spectra, predicted mass spectra or previously acquired mass spectra. An example of spectra similarity scores are counting-based scores (or peak presence/absence-based similarity/dissimilarity scores). Such scores may include determining how many fragment ions match between the two spectra. Spectrum similarity scores may also include measures of normalized spectrum contrast angle (SA) between the intensities of two spectra. The calculation of spectra similarity scores such as SA would be well known to the skilled person (for example as set out in Toprak UH et al. "Conserved peptide fragmentation as a benchmarking tool for mass spectrometers and a discriminating feature for targeted proteomics." Mol Cell Proteomics. 2014 Aug;13(8):2056-71. doi: 10.1074/mcp.O113.036475 and Wan KX et al. "Comparing similar spectra: from similarity index to spectral contrast angle." J Am Soc Mass Spectrom. 2002 Jan;13(1):85-8. doi: 10.1016/S1044-0305(01)00327-0) and is not discussed further herein.

However, if the experimental spectrum is chimeric, such scores are influenced by all analytes underlying the experimental spectrum and bias the resulting scores. The calculated mass spectra coefficients set out above enable the calculation of improved spectra similarity scores. For example a further mass spectrum may be generated from the experimental mass spectrum by subtracting the contributions of all of the candidate mass spectra, each scaled with the corresponding mass spectra coefficient, with the exception of a given candidate mass spectrum. In other words, the coefficients of the candidates can be used to predict the proportional intensity of all but the given candidate spectrum, add the proportional intensities together and subsequently subtract said sum from the experimental spectrum to calculate the further mass spectrum. This further mass spectrum may be thought of as the experimental spectrum with the contributions of all interfering spectra removed. In effect, the further mass spectrum simulates a situation where the precursor molecular species of the given candidate mass spectrum would be the sole analyte in a mass spectrum. A spectral similarity score may then be calculated between the further mass spectrum and the given candidate mass spectrum. In this way it will be appreciated that the resulting spectral similarity score is improved as bias resulting from the other analytes present may be reduced.

It will be appreciated that the above-described systems may also be used for molecular species derivatized with MS2/MSn labile isobaric labelling reagents (e.g. TMT). The molecular species in a given sample 100 may be labelled with a tagging reagent including a reporter group that produces a reporter ion upon fragmentation. Multiple samples labelled with different tagging reagents can then be combined. The differentially labelled identical precursor molecular species may be isolated together and fragmented to obtain an experimental mass spectrum including one unique reporter ion per sample (or per tagging reagent). The generated reporter ions are indicative of the relative ratio of the precursor molecular species of the combined samples prior to pooling of the samples and can be used for quantification.

It will be understood that if the experimental mass spectrum is chimeric, such reporter ion intensities may be influenced by all molecular species represented in the experimental mass spectrum. As such, the quantification of a given molecular species may be biased due to multiple molecular species in the sample contributing to the intensity of the same reporter ion. The calculated mass spectra coefficients set out above enable the calculation of improved quantitative values for the molecular species.

For example, the optimized mass spectra coefficients may be used to derive a chimericity criterion to select/deselect mass spectra regarded for quantification.

Additionally or alternatively, the calculated mass spectra coefficients may be used to distribute the experimental reporter ion intensities to the underlying molecular species according to their contributions to the chimeric spectrum by solving a system of linear equations consisting of the corresponding mass spectra coefficients and the mixing ratios for the different samples obtained from other received mass spectra. In this way it will be appreciated that the resulting quantitative information from the received mass spectrum is improved as bias resulting from the other molecular species present may be reduced.

As an example in a scenario where there are three experimental spectra: two pure spectra generated from peptide A and peptide B, respectively, and one chimeric spectrum generated from peptide A, peptide B and peptide C. In order to calculate the reporter-ion intensities for peptide C in the presence of bias from peptides A and B in the chimeric spectrum, it is possible to multiply the reporter-ion intensities of peptide A and peptide B from their corresponding pure spectra with the corresponding optimized coefficients obtained by applying the above-described methods to the chimeric mass spectrum. By subtracting these scaled reporter ion intensities from the reporter-ion intensities of the chimeric spectrum, a reporter ion intensity for peptide C is produced with the bias from peptides A and B substantially eliminated.

It will be appreciated that the above-described systems may also be used with existing target-decoy techniques as known in the art. Such techniques often involve allowing a number of decoy mass spectra to compete with the fragment mass spectra when attempting to identify fragment mass spectra in experimental mass spectrometry data. The decoy mass spectra are usually suitably scrambled candidate mass spectra where the mass channels for the intensity peaks have been altered. As such decoy mass spectra do not correspond to "real" precursor molecular species and any match between the experimental mass spectrometry data and a decoy mass spectrum must represent a false positive (or false discovery). As such the number of such decoy matches may be used to estimate the false discovery rate for given experimental mass spectrometry data 390. This can therefore be used to estimate a false positive likelihood (or other correctness score) for any matches to non-decoy fragment spectra. Target decoy techniques in general are known in the art - see for example Elias, J., Haas, W., Faherty, B. et al. "Comparative evaluation of mass spectrometry platforms used in large-scale proteomics investigations." Nat Methods 2, 667-675 (2005). DOI: 10.1038/nmeth785; Elias JE, Gygi SP. "Target-decoy search strategy for mass spectrometry-based proteomics." Methods Mol Biol. 2010;604:55-71. doi: 10.1007/978-1-60761-444-9_5; and "Reverse and Random Decoy Methods for False Discovery Rate Estimation in High Mass Accuracy Peptide Spectral Library Searches" Zheng Zhang, et al. Journal of Proteome Research 2018 17 (2), 846-857, DOI: 10.1021/acs.jproteome.7b00614.

In this way it will be understood that the candidate selection module 420 of systems 400 and 600 may be arranged to include one or more decoy mass spectra in the set of candidate mass spectra. The decoy mass spectra may be obtained from a suitable mass spectra database 425. Additionally, or alternatively the candidate selection module 420 may be arranged to generate one or more decoy mass spectra based on one or more existing candidate mass spectra. This may be done using existing known techniques for generating decoy mass spectra. The use of theoretical mass spectra as candidate mass spectra as discussed above is particularly advantageous as decoy mass spectra having realistic intensity distributions can more easily be generated.

It will be appreciated that the methods described have been shown as individual steps carried out in a specific order. However, the skilled person will appreciate that these steps may be combined or carried out in a different order whilst still achieving the desired result.

It will be appreciated that embodiments of the invention may be implemented using a variety of different information processing systems. In particular, although the figures and the discussion thereof provide an exemplary computing system and methods, these are presented merely to provide a useful reference in discussing various aspects of the invention. Embodiments of the invention may be carried out on any suitable data processing device, such as a personal computer, laptop, personal digital assistant, mobile telephone, set top box, television, server computer, etc. Of course, the description of the systems and methods has been simplified for purposes of discussion, and they are just one of many different types of systems and methods that may be used for embodiments of the invention. It will be appreciated that the boundaries between logic blocks are merely illustrative and that alternative embodiments may merge logic blocks or elements, or may impose an alternate decomposition of functionality upon various logic blocks or elements.

It will be appreciated that the above-mentioned functionality may be implemented as one or more corresponding modules as hardware and/or software. For example, the above-mentioned functionality may be implemented as one or more software components for execution by a processor of the system. Alternatively, the above-mentioned functionality may be implemented as hardware, such as on one or more field-programmable-gate-arrays (FPGAs), and/or one or more application-specific-integrated-circuits (ASICs), and/or one or more digital-signal-processors (DSPs), and/or other hardware arrangements. Method steps implemented in flowcharts contained herein, or as described above, may each be implemented by corresponding respective modules; multiple method steps implemented in flowcharts contained herein, or as described above, may be implemented together by a single module.

It will be appreciated that, insofar as embodiments of the invention are implemented by a computer program, then a storage medium and a transmission medium carrying the computer program form aspects of the invention. The computer program may have one or more program instructions, or program code, which, when executed by a computer carries out an embodiment of the invention. The term "program" as used herein, may be a sequence of instructions designed for execution on a computer system, and may include a subroutine, a function, a procedure, a module, an object method, an object implementation, an executable application, an applet, a servlet, source code, object code, a shared library, a dynamic linked library, and/or other sequences of instructions designed for execution on a computer system. The storage medium may be a magnetic disc (such as a hard drive or a floppy disc), an optical disc (such as a CD-ROM, a DVD-ROM or a BluRay disc), or a memory (such as a ROM, a RAM, EEPROM, EPROM, Flash memory or a portable/removable memory device), etc. The transmission medium may be a communications signal, a data broadcast, a communications link between two or more computers, etc.

**Claims**

1. A method of identifying one or more molecular species represented in mass spectrometry data, the method comprising:

   obtaining a set of candidate mass spectra for the mass spectrometry data, wherein each candidate mass spectrum corresponds to a respective candidate molecular species;
   optimizing a set of mass spectra coefficients for the set of candidate mass spectra, based on the mass spectrometry data, wherein said optimizing comprises:

   varying the mass spectra coefficient values based on an objective function,
   wherein the objective function relates a linear combination of the candidate mass spectra, according to the mass spectra coefficients, to the mass spectrometry data,

   subject to a regularization term of the objective function constraining the number of non-zero mass spectra coefficient values;
   providing, for one or more of the candidate molecular species, a respective indication of a match in the mass spectrum, based at least in part on the optimized set of mass spectra coefficients.

2. The method of claim 1 wherein the one or more molecular species are one or more precursor molecular species, the mass spectrometry data is a fragment mass spectrum derived from the one or more precursor molecular species, and each candidate mass spectrum is a candidate fragment mass spectrum corresponding to a respective candidate molecular species.

3. The method of claim 2 wherein the step of providing comprises identifying the one or more candidate molecular species as sample molecular species represented in a chimeric mass spectrum based on the optimized set of fragment mass spectra coefficients.

4. The method of claim 2 or 3 wherein the precursor molecular species represented in one or more candidate fragment mass spectra are peptides.

5. The method of claim 1 wherein the mass spectrometry data is an MS1 mass spectrum of the one or more molecular species and each candidate mass spectrum comprises an isotope pattern corresponding to a respective candidate molecular species.

6. The method of any one of claims 1 or 5 wherein the mass spectrometry data is an MS1 mass spectrum and wherein one or more of the candidate mass spectra are selected as part of the set of candidate mass spectra based on an MS2 mass spectrum corresponding to the MS1 mass spectrum.

7. The method of any one of the preceding claims wherein for a given candidate mass spectrum:
   a spectrum similarity score is calculated between the given candidate mass spectrum and a further mass spectrum, wherein the further mass spectrum is generated by subtracting each of the other candidate mass spectra from the mass spectrometry data according to the optimized set of mass spectra coefficients.

8. The method of any preceding claim wherein the respective indication comprises a quantity of the corresponding candidate molecular species present in the mass spectrum.

9. The method of any preceding claim further comprising identifying the mass spectrometry data as a chimeric mass spectrum based on the optimized set of mass spectra coefficients.

10. The method of any one of claims 1-4 wherein the one or more molecular species are a plurality of isobarically labelled precursor molecular species, the mass spectrometry data is a fragment mass spectrum derived from the plurality of isobarically labelled precursor molecular species, and each candidate mass spectrum is a candidate fragment mass spectrum corresponding to a respective candidate isobarically labelled molecular species;
    wherein the step of providing further comprises generating reporter ion intensities for one or more of candidate mass spectra and generating corrected reporter ion intensities for at least one of the precursor molecular species based on a difference between a reporter ion intensity for the precursor molecular species generated from the mass spectrometry data and the reporter ion intensities for the one or more candidate mass spectra scaled by the corresponding optimized mass spectra coefficients.

11. The method of any preceding claim wherein the mass spectrometry data is part of a series of mass spectrometry data for a separation parameter wherein the regularization term comprises a constraint enforcing a relation between the mass spectra coefficient for a given candidate mass spectrum and a mass spectra coefficient of the same candidate mass spectrum determined for further mass spectrometry data of the series.

12. The method of any preceding claim wherein the regularization term comprises an $L_1$ norm of the mass spectra coefficient values, wherein optionally the regularization term comprises an $L_2$ norm of the mass spectra coefficient values.

13. The method of any preceding claim wherein the optimizing step further comprises varying a parameter specifying the degree of regularization of the regularization term.

14. The method of any preceding claim wherein the objective function provides a measure of difference between the linear combination of the candidate mass spectra and the mass spectrometry data, optionally wherein the step of varying is carried out with the aim of obtaining an extremum value of the objective function.

15. A system arranged to carry out a method according to any one of the preceding claims.

16. A computer program which, when executed by a processor, causes the processor to carry out a method according to any one of claims 1-14.

17. A computer-readable medium storing a computer program according to claim 16.

Fig 1a

EP 4 102 509 A1

190

100

101

200

205

205

205

201

290

Fig 1b

Fig 1c

Fig 2

Fig 3

EP 4 102 509 A1

**480**

```
        ┌──────────────────────┐
        │ 482                  │
        │                      │
        └──────────┬───────────┘
                   │
                   ▼
        ┌──────────────────────┐
        │ 484                  │
        │   ┌───────────────┐  │
        │   │ 486           │  │
        │   └───────────────┘  │
        └──────────┬───────────┘
                   │
                   ▼
        ┌──────────────────────┐
        │ 488                  │
        │                      │
        └──────────┬───────────┘
                   │
                   ▼
```

**Fig 4**

**Fig 5**

Fig 6

EP 4 102 509 A1

Fig 7

| Modified Sequence | Spectral similarity | PSM Score | Coefficient | PSM Q-Value |
|---|---|---|---|---|
| LYVDFPQHRL | 0.956 | 1.491 | 0.997 | 0.00002 |
| IVLELDR | 0.897 | 0.193 | 0.244 | 0.00243 |
| AVVWDLR | 0.786 | 0.106 | 0.312 | 0.0046 |
| LVLQMPR | 0.791 | 0.044 | 0.112 | 0.007 |

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RYAN PECKNER ET AL: "Specter: linear deconvolution for targeted analysis of data-independent acquisition mass spectrometry proteomics", NATURE METHODS, vol. 15, no. 5, 2 April 2018 (2018-04-02), pages 371-378, XP055670100, New York ISSN: 1548-7091, DOI: 10.1038/nmeth.4643 * the whole document * | 1-17 | INV. G16C20/20 |
| A | US 2014/138537 A1 (GROTHE JR ROBERT A [US] ET AL) 22 May 2014 (2014-05-22) * the whole document * | 1-17 | |
| A | ZHANG WENJU ET AL: "ChimST: An Efficient Spectral Library Search Tool for Peptide Identification from Chimeric Spectra in Data-Dependent Acquisition", IEEE/ACM TRANSACTIONS ON COMPUTATIONAL BIOLOGY AND BIOINFORMATICS, IEEE SERVICE CENTER, NEW YORK, NY, US, vol. 18, no. 4, 7 October 2019 (2019-10-07), pages 1416-1425, XP011870914, ISSN: 1545-5963, DOI: 10.1109/TCBB.2019.2945954 [retrieved on 2021-08-05] * the whole document * | 1-17 | |
| A | EP 3 002 696 A1 (THERMO FINNIGAN LLC [US]) 6 April 2016 (2016-04-06) * the whole document * | 1-17 | TECHNICAL FIELDS SEARCHED (IPC) G16C |

The present search report has been drawn up for all claims

| Place of search | Date of completion of the search | Examiner |
|---|---|---|
| The Hague | 16 November 2021 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 8053

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2021

| Patent document cited in search report | Publication date | Patent family member(s) | Publication date |
|---|---|---|---|
| US 2014138537 A1 | 22-05-2014 | EP 2741224 A1<br>US 2014138535 A1<br>US 2014138537 A1 | 11-06-2014<br>22-05-2014<br>22-05-2014 |
| EP 3002696 A1 | 06-04-2016 | NONE | |

EPO FORM P0459

For more details about this annex : see Official Journal of the European Patent Office, No. 12/82

## REFERENCES CITED IN THE DESCRIPTION

**Patent documents cited in the description**

- WO 02078046 A

**Non-patent literature cited in the description**

- **FEDOR KRYUCHKOV et al.** Deconvolution of Mixture Spectra and Increased Throughput of Peptide Identification by Utilization of Intensified Complementary Ions Formed in Tandem Mass Spectrometry. *Journal of Proteome Research,* 2013, vol. 12 (7), 3362-3371
- **TSOU, CC et al.** DIA-Umpire: comprehensive computational framework for data-independent acquisition proteomics. *Nature Methods,* 2015, vol. 12, 258-264
- **GESSULAT, S. ; SCHMIDT, T. ; ZOLG, D.P. et al.** Prosit: proteome-wide prediction of peptide tandem mass spectra by deep learning. *Nat Methods,* 2019, vol. 16, 509-518
- **ZHOU et al.** pDeep: Predicting MS/MS Spectra of Peptides with Deep Learning. *Analytical Chemistry,* 2017, vol. 89 (23), 12690-12697
- **SVEN DEGROEVE ; LENNART MARTENS.** MS2PIP: a tool for MS/MS peak intensity prediction. *Bioinformatics,* 15 December 2013, vol. 29 (24), 3199-3203
- **ROVSHAN G. SADYGOV.** Poisson Model To Generate Isotope Distribution for Biomolecules. *Journal of Proteome Research,* 2018, vol. 17 (1), 751-758
- **DITTWALD et al.** BRAIN: A Universal Tool for High-Throughput Calculations of the Isotopic Distribution for Mass Spectrometry. *Analytical Chemistry,* 2013, vol. 85 (4), 1991-1994

- **DITTWALD, P et al.** BRAIN 2.0: Time and Memory Complexity Improvements in the Algorithm for Calculating the Isotope Distribution. *J. Am. Soc. Mass Spectrom.,* 2014, vol. 25, 588-594
- **MARTIN LOOS et al.** Accelerated Isotope Fine Structure Calculation Using Pruned Transition Trees. *Analytical Chemistry,* 2015, vol. 87 (11), 5738-5744
- **PATRICK KREITZBERG et al.** Fast Exact Computation of the k Most Abundant Isotope Peaks with Layer-Ordered Heaps. *Analytical Chemistry,* 2020, vol. 92 (15), 10613-10619
- **TOPRAK UH et al.** Conserved peptide fragmentation as a benchmarking tool for mass spectrometers and a discriminating feature for targeted proteomics. *Mol Cell Proteomics,* August 2014, vol. 13 (8), 2056-71
- **WAN KX et al.** Comparing similar spectra: from similarity index to spectral contrast angle. *J Am Soc Mass Spectrom,* January 2002, vol. 13 (1), 85-8
- **ELIAS, J. ; HAAS, W. ; FAHERTY, B. et al.** Comparative evaluation of mass spectrometry platforms used in large-scale proteomics investigations. *Nat Methods,* 2005, vol. 2, 667-675
- **ELIAS JE ; GYGI SP.** Target-decoy search strategy for mass spectrometry-based proteomics. *Methods Mol Biol,* 2010, vol. 604, 55-71
- **ZHENG ZHANG et al.** Reverse and Random Decoy Methods for False Discovery Rate Estimation in High Mass Accuracy Peptide Spectral Library Searches. *Journal of Proteome Research,* 2018, vol. 17 (2), 846-857